(19) Europäisches Patentamt
European Patent Office
Office européen des brevets

(11) Veröffentlichungsnummer: **0 181 282**
**A1**

(12) # EUROPÄISCHE PATENTANMELDUNG

(21) Anmeldenummer: 85810438.3

(22) Anmeldetag: 25.09.85

(51) Int. Cl.$^4$: **C 07 D 487/04**
A 61 K 31/505, A 61 K 31/41
//(C07D487/04, 249:00, 239:00)

(30) Priorität: 01.10.84 US 655831

(43) Veröffentlichungstag der Anmeldung:
14.05.86 Patentblatt 86/20

(84) Benannte Vertragsstaaten:
AT BE CH DE FR GB IT LI LU NL SE

(71) Anmelder: CIBA-GEIGY AG
Klybeckstrasse 141
CH-4002 Basel(CH)

(72) Erfinder: Francis, John, E, Dr.
Palmer Lane West
Pleasantville New York 10570(US)

(72) Erfinder: Gelotte, Karl O.
106 Stanie Brae Drive
Watchung, N.J. 07060(US)

(54) **Triazolochinazolinverbindungen.**

(57) Verbindungen der Formel I

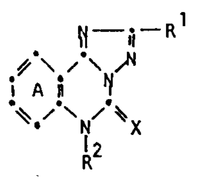

(I)

sowie Salze davon werden offenbart, worin $R^1$, $R^2$, X und Ring A die in der Beschreibung angegebenen Bedeutungen haben, ferner Verfahren zu ihrer Herstellung, pharmazeutische Zusammensetzungen, die diese Verbindungen enthalten, und ihre Verwendung als Anxiomodulatoren und Benzodiazepinantagonisten.

EP 0 181 282 A1

Croydon Printing Company Ltd.

0181282

CIBA-GEIGY AG
Basel (Schweiz)

4-15093/+/CGC 1101

# Triazolochinazolinverbindungen

Die Erfindung betrifft neue [1,2,4]Triazolo[1,5-c]chinazolinverbindungen der Formel I,

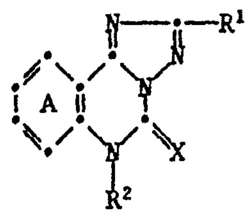

(I)

worin $R^1$ Phenyl oder durch Niederalkyl, Niederalkoxy, Hydroxy, Halogen oder Trifluormethyl substituiertes Phenyl bedeutet, oder einen 5-gliedrigen aromatischen oder teilweise oder vollständig gesättigten heterocyclischen Ring, der 1 bis 3 Heteroatome aus der Gruppe N, O und S enthält oder einen 6-gliedrigen aromatischen oder teilweise oder vollständig gesättigten heterocyclischen Ring, der 1 bis 4 Heteroatome aus der Gruppe N, O und S enthält, wobei dieser heterocyclische Ring an den Triazolochinazolinkern über ein Kohlenstoffatom gebunden ist, und wobei dieser heterocyclische Ring unsubstituiert oder durch Niederalkyl, Hydroxy, Amino, Halogen oder Hydroxyniederalkyl substituiert sein kann, $R^2$ Wasserstoff, Niederalkyl, Hydroxyniederalkyl, Niederalkenyl, Arylniederalkyl, Arylniederalkenyl oder Aryl bedeutet, Ring A unsubstituiert oder durch Niederalkyl, Niederalkoxy, Hydroxy, Halogen, Trifluormethyl, Nitro, Amino, Niederalkylthio, Niederalkylsulfinyl, Niederalkylsulfonyl oder Arylniederalkoxy substituiert ist, und worin X Sauerstoff, Schwefel oder N-$R^3$ bedeutet, worin $R^3$ für Wasserstoff, Niederalkyl, Arylniederalkyl, Cycloalkyl, Niederalkenyl, worin die Doppelbindung

vom Stickstoffatom durch mindestens ein gesättigtes Kohlenstoffatom getrennt ist, Niederalkinyl, worin die Dreifachbindung vom Stickstoffatom durch mindestens ein gesättigtes Kohlenstoffatom getrennt ist, Aryl, Aminoniederalkyl, Niederalkylaminoniederalkyl, Diniederalkylaminoniederalkyl oder Hydroxyniederalkyl steht, Salze und Tautomere dieser Verbindungen, Verfahren zu deren Herstellung, pharmazeutische Zusammensetzungen, die diese Verbindungen enthalten, und die Verwendung solcher Verbindungen und pharmazeutischen Zusammensetzungen, die diese Verbindungen enthalten.

Wenn $R^2$ Wasserstoff bedeutet, können die Verbindungen der Formel I in einer tautomeren Form vorliegen, wie in Formel I' dargestellt:

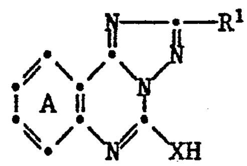

(I').

Der Ausdruck "nieder" in Verbindung mit organischen Gruppen und Substituenten steht für Gruppen und Substituenten mit bis zu 7 Kohlenstoffatomen und vorzugsweise für solche mit bis zu 4 Kohlenstoffatomen.

Cycloalkyl enthält 3 bis 20, vorzugsweise 5 bis 8 und insbesondere 6 Ringglieder und kann beispielsweise Cyclopropyl, Cyclopentyl, Cyclohexyl, Cycloheptyl oder Cyclooctyl sein. Cycloalkylgruppen können durch eine oder mehrere zusätzliche Gruppen wie beispielsweise Niederalkyl- oder Arylgruppen substituiert sein.

Arylgruppen können durch eine oder mehrere zusätzliche Gruppen substituiert sein wie beispielsweise durch Niederalkyl, Halogen, Hydroxy, Nitro, Amino, Amino das durch ein oder mehrere Niederalkylgruppen substituiert ist oder Aryl.

Eine Niederalkylgruppe kann unsubstituiert oder substituiert sein und ist z.B. Methyl, Ethyl, Propyl, Isopropyl, n-Butyl, t-Butyl, Pentyl, Neopentyl, Isopentyl oder Heptyl. Die bevorzugte Gruppe ist Methyl.

Geeignete Substituenten für Niederalkylgruppen schliessen Halogen und Hydroxy ein. Substituierte Niederalkylgruppen sind beispielsweise Chlormethyl, Dibrommethyl, Trifluormethyl, Trichlormethyl und Hydroxymethyl.

Niederalkoxy ist z.B. Methoxy, Ethoxy, Propoxy, Isopropoxy, n-Butoxy, t-Butoxy, Pentoxy, Isopentoxy, Hexyloxy und Heptyloxy. Bevorzugt ist Methoxy.

Halogen ist z.B. Fluor oder Brom oder vorzugsweise Chlor.

Ein 5-gliedriger heterocyclischer Ring, der 1 bis 3 Heteroatome aus der Gruppe N, O und S enthält, enthält vorzugsweise 1 oder 2 Heteroatome aus der Gruppe N, O und S. Insbesondere enthalten die Ringe 1 bis 3 Stickstoffatome; ein Sauerstoffatom; ein Schwefelatom; oder ein Stickstoffatom und ein Sauerstoff- oder ein Schwefelatom. Entsprechende heterocyclische Ringe können aromatisch sein, typische Vertreter sind beispielsweise Thienyl, Furyl, Pyrrolyl, Thiazolyl, Isothiazolyl, Oxazolyl, Imidazolyl, Pyrazolyl, 1,2,3- oder 1,2,4-Triazolyl.

Ferner kann ein 5-gliedriger heterocyclischer Ring, der Heteroatome wie vorstehend definiert enthält, teilweise oder vollständig gesättigt sein, ein solcher Ring ist beispielsweise Pyrrolinyl, Pyrrolidinyl, Dihydrofuranyl, Tetrahydrofuranyl, Dihydrothienyl, Tetrahydrothienyl, Pyrazolinyl, Thiazolinyl, Oxazolinyl und Triazolinyl.

Ein 6-gliedriger heterocyclischer Ring, der 1 bis 4 Heteroatome aus der Gruppe N, O und S enthält, kann Heteroatome wie vorstehend für die 5-gliedrigen Ringe definiert enthalten. Er kann aromatisch sein, z.B. Pyridyl, oder nichtaromatisch, z.B. Pyranyl, Piperidinyl, Morpholinyl und O-Ribofuranosyl.

Aryl bedeutet einen aromatischen heterocyclischen Ring, der wie vorstehend definiert ist, oder z.B. Phenyl.

Arylniederalkyl ist z.B. Benzyl, Phenylethyl, Thenyl, Thienylethyl, Furfuryl, Furylethyl, Pyridylmethyl, Pyridylethyl, Pyrrolylmethyl, Pyrrolylethyl, Piperidinylmethyl und Piperidinylethyl.

Arylniederalkenyl ist z.B. Cinnamyl, 3-(2- oder 3-Furyl)-2-propen-1-yl, 3-(2- oder 3-Pyrrolyl)-2-propen-1-yl oder 3-(2-, 3- oder 4-Pyridyl)-2-propen-1-yl.

Niederalkylthio ist z.B. Methylthio, Ethylthio oder Isopropylthio. Niederalkylsulfinyl ist z.B. Methylsulfinyl oder Ethylsulfinyl. Niederalkylsulfonyl ist z.B. Methylsulfonyl oder Ethylsulfonyl.

Niederalkenyl kann z.B. Ethenyl, Propenyl oder Butenyl sein. Niederalkinyl kann z.B. Ethinyl oder Propinyl sein.

Aminoniederalkyl kann z.B. 2-Aminoethyl oder 2- oder 3-Aminopropyl sein. Niederalkylaminoniederalkyl kann z.B. 2-(N-Methylamino)-ethyl oder 2-(N-Ethylamino)-ethyl sein. Diniederalkylaminoniederalkyl kann z.B. 2-(N,N-Dimethylamino)-ethyl oder 2-(N,N-Diethylamino)-ethyl sein.

Die beanspruchten Verbindungen können Säureadditionssalze bilden, insbesondere pharmazeutisch annehmbare Säureadditionssalze. Pharmazeutisch annehmbare Salze sind Säureadditionssalze, die vorzugsweise von therapeutisch annehmbaren anorganischen oder organischen Säuren gebildet werden, wie von starken Mineralsäuren, z.B. Halogenwasserstoffsäuren, wie Chlorwasserstoff- oder Bromwasserstoffsäure;

Schwefelsäure, Phosphor- oder Salpetersäure; aliphatische oder aromatische Carbonsäuren oder Sulfonsäuren, z.B. Ameisensäure, Essigsäure, Propionsäure, Bernsteinsäure, Glykolsäure, Milchsäure, Aepfelsäure, Weinsäure, Glukonsäure, Zitronensäure, Maleinsäure, Fumarsäure, Zimtsäure, Brenztraubensäure, Phenylessigsäure, Benzoesäure, 4-Aminobenzoesäure, Anthranilsäure, 4-Hydroxybenzoesäure, Salicylsäure, 4-Aminosalicylsäure, Pamoasäure, Nikotinsäure, Methansulfonsäure, Ethansulfonsäure, Hydroxyethansulfonsäure, Benzolsulfonsäure, p-Toluolsulfonsäure, Naphthalinsulfonsäure, Sulfanilsäure, Cyclohexylsulfaminsäure oder Ascorbinsäure.

Die Verbindungen der Formel I und ihre pharmazeutisch annehmbaren Salze sind Benzodiazepinantagonisten und Anxiomodulatoren. Ferner sind diese Verbindungen Adenosinantagonisten und können Antiasthmamittel und das zentrale Nervensystem stimulierende Mittel sein und können das Erinnerungsvermögen verbessern. Diese nützlichen Eigenschaften lassen sich an Säugern wie Menschen demonstrieren, wenn die Verbindungen oral, intraperitoneal oder durch Inhalation in Dosen von 0,01 mg/kg bis 500 mg/kg Körpergewicht, vorzugsweise 0,1 bis 100 mg/kg und ganz bevorzugt 10 bis 30 mg/kg Körpergewicht gegeben werden.

Die Erfindung bezieht sich besonders auf Verbindungen der Formel I, worin $R^1$ für Phenyl steht oder für Phenyl, das durch 1 bis 3 Reste der Art Niederalkyl, Niederalkoxy, Hydroxy, Halogen oder Trifluormethyl substituiert ist; oder $R^1$ bedeutet einen 5-gliedrigen aromatischen oder teilweise oder vollständig gesättigten heterocyclischen Ring, der 1 bis 3 Heteroatome aus der Gruppe N, O und S enthält oder einen 6-gliedrigen aromatischen oder teilweise oder vollständig gesättigten heterocyclischen Ring, der 1 bis 4 Heteroatome aus der Gruppe N, O und S enthält, wobei dieser heterocyclische Ring an den Triazolochinazolinkern über ein Kohlenstoffatom gebunden ist, und wobei dieser heterocyclische Ring unsubstituiert oder durch Niederalkyl, Hydroxy oder Halogen substituiert sein kann; worin $R^2$ für Wasserstoff, Niederalkyl, Arylniederalkyl, Niederalkenyl, Arylniederalkenyl oder Aryl steht, worin X O, S oder N-$R^3$

bedeutet, wobei $R^3$ für Wasserstoff, Niederalkyl, Arylniederalkyl, Aminoniederalkyl oder Niederalkylaminoniederalkyl steht; und worin Ring A unsubstituiert ist oder durch ein bis drei Gruppen der Art Niederalkyl, Niederalkoxy, Hydroxy, Halogen oder Trifluormethyl substituiert ist; und pharmazeutisch annehmbare Salze davon.

Die Erfindung bezieht sich insbesondere auf Verbindungen der Formel I, worin $R^1$ für Phenyl steht oder für Phenyl, das durch ein bis drei Reste der Art Niederalkyl, z.B. Methyl oder Ethyl, Niederalkoxy, z.B. Methoxy, Hydroxy, Halogen, z.B. Fluor oder Chlor, und Trifluormethyl substituiert ist; oder für einen 5-gliedrigen aromatischen heterocyclischen Ring, der 1 bis 3 Heteroatome aus der Gruppe N, O und S enthält oder für einen 6-gliedrigen aromatischen heterocyclischen Ring, der 1 bis 4 Heteroatome aus der Gruppe N, O und S enthält, wobei ein solcher heterocyclischer Ring über ein Kohlenstoffatom gebunden ist; z.B. 2- oder 3-Thienyl, 2- oder 3-Furyl, 2- oder 3-Pyrrolyl, 2-, 3- oder 4-Pyridyl; 3- oder 4-Pyrazolyl oder 2- oder 4-Imidazolyl; wobei dieser aromatische heterocyclische Ring unsubstituiert oder durch Hydroxy, Niederalkyl, z.B. Methyl oder Ethyl, oder Halogen, z.B. Fluor oder Chlor, substituiert sein kann; worin $R^2$ für Wasserstoff oder Niederalkyl, z.B. Methyl steht; worin X Sauerstoff bedeutet, insbesondere wenn $R^1$ Phenyl oder substituiertes Phenyl bedeutet; oder worin X $N-R^3$ bedeutet, worin $R^3$ für Wasserstoff oder Niederalkyl, z.B. Methyl oder Ethyl steht, insbesondere wenn $R^1$ einen aromatischen heterocyclischen Ring oder einen substituierten aromatischen heterocyclischen Ring bedeutet; und worin Ring A unsubstituiert ist oder substituiert durch 1 bis 3 Gruppen der Art Halogen, z.B. 8- oder 9-Fluor, 8- oder 9-Chlor, oder 7,9-Dichlor; oder Niederalkyl, z.B. 8- oder 9-Methyl, sowie pharmazeutisch annehmbare Salze davon.

Die Erfindung bezieht sich ganz besonders auf Verbindungen der Formel I, worin $R^1$ für Phenyl oder für durch Halogen substituiertes Phenyl steht, insbesondere in der ortho- oder meta-Position, insbesondere durch Fluor oder Chlor; oder für Furyl insbesondere 2-Furyl; worin $R^2$ Wasserstoff bedeutet; worin X für Sauerstoff

steht, insbesondere, wenn $R^1$ Phenyl, substituiert durch Halogen, z.B. Fluor in ortho- oder meta-Position, bedeutet; oder worin X für NH steht, insbesondere wenn $R^1$ 2-Furyl bedeutet; und worin Ring A durch Halogen in der 8- oder 9-Stellung substituiert ist, insbesondere durch Chlor in der 9-Stellung, und pharmazeutisch annehmbare Salze davon.

Die Erfindung bezieht sich insbesondere auf die spezifisch in den Beispielen erwähnten Verbindungen.

Die Verbindungen der Formel I können auf an sich bekannte Weise hergestellt werden, z.B.

a) durch Behandeln einer Verbindung der Formel II,

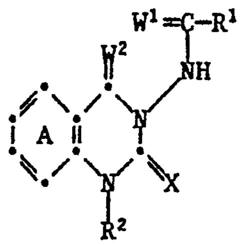

(II)

worin $R^1$, $R^2$, X und Ring A wie vorstehend definiert sind, einer der beiden Reste $W^1$ und $W^2$ für NH und der andere der beiden Reste $W^1$ und $W^2$ für O oder NH steht, mit einer Base, oder

b) durch Behandeln einer Verbindung der Formel III,

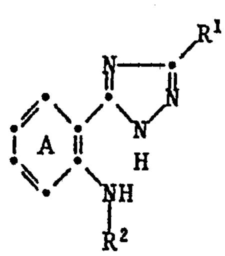

(III)

worin $R^1$, $R^2$ und Ring A wie vorstehend definiert sind, mit einem reaktiven Derivat der Kohlensäure, oder

c) durch Behandeln einer Verbindung der Formel IV,

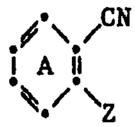

(IV)

worin Ring A wie vorstehend definiert ist und Z das Radikal eines Kohlensäurederivates, das über ein Stickstoffatom gebunden ist, bedeutet, mit einem Hydrazid der Formel V,

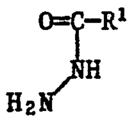

(V)

oder d), um eine Verbindung der Formel I, worin $R^2$ für Wasserstoff und X für Sauerstoff steht, zu erhalten, durch Behandeln einer Verbindung der Formel VI,

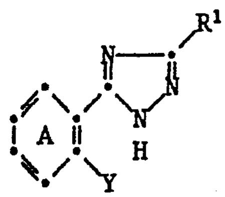

(VI)

worin Y für eine Gruppe steht, die sich durch ein Oxydationsmittel in die Gruppe -N=C=O überführen lässt, mit einem solchen Oxidationsmittel gefolgt von einem Ringschluss, oder

e), um eine Verbindung der Formel I zu erhalten, worin $R^2$ für Wasserstoff steht, durch Ueberführen der Gruppe L in einer Verbindung der Formel VII,

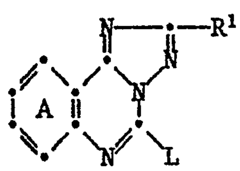

(VII)

worin L aus der Gruppe Halogen, Niederalkoxy, Arylniederalkoxy, Mercapto, Niederalkylthio und Isothiocyanato ausgewählt ist, in die Gruppe XH, oder,

f), um eine Verbindung der Formel I zu erhalten, worin $R^2$ für Wasserstoff steht, durch Behandeln einer Verbindung der Formel VIII

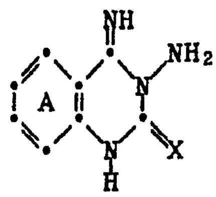

(VIII)

mit einem reaktiven Derivat einer Carbonsäure der Formel $R^1$-COOH, und wenn erwünscht, durch Ueberführung einer erhaltenen Verbindung in eine andere Verbindung der Erfindung und/oder durch Ueberführen eines erhaltenen Salzes in die freie Verbindung oder in ein anderes Salz und/oder durch Ueberführen einer erhaltenen freien Verbindung, die eine salzbildende Gruppe aufweist, in ein Salz.

Verfahren a): Die Base ist z.B. eine organische Base, vorzugweise ein tertiäres Amin, z.B. Pyridin oder Triethylamin, kann aber auch Ammoniak sein.

Die Ammoniakquelle kann z.B. ein Carbamat sein, wie ein Niederalkylcarbamat, z.B. Methyl- oder Ethylcarbamat oder Ammoniumcarbonat. Die Umsetzungen können mit oder ohne inertes Lösungsmittel, bei atmosphärischem oder erhöhtem Druck, z.B. in einem Autoklaven, durchgeführt werden.

Verbindungen der Formel II können hergestellt werden, indem eine Verbindung der Formel IX,

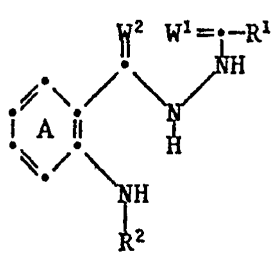

(IX)

worin $W^1$ und $W^2$ unabhängig voneinander O oder NH bedeuten, mit Ammoniak und/oder mit einem reaktiven Derivat der Kohlensäure umgesetzt werden.

Die reaktiven Derivate der Kohlensäure umfassen Ester, Amide und Anhydride der Kohlensäure, wie auch die entsprechenden Thio- oder Iminoverbindungen, wie z.B. Phosgen, Kohlensäurediethylester, Thiophosgen, Chlorkohlensäuretrichlormethylester, O-Ethylurethan, Harnstoff, Cyanamid oder Guanidin.

Die Ammoniakquelle kann gleichzeitig auch als reaktives Derivat der Kohlensäure dienen, wie z.B. ein O-Niederalkylurethan, wie O-Ethylurethan.

Wenn $W^1$ und $W^2$ Sauerstoff bedeuten, wird die Verbindung der Formel IX aus einem Anthranilsäureester und einem Hydrazid der Formel V ($R^1CONHNH_2$) hergestellt. Wenn $W^1$ und $W^2$ NH bedeuten, wird die Verbindung der Formel IX aus einem Anthranilonitril und einem Hydrazidin der Formel $R^1C(=NH)NHNH_2$ oder einem Hydrazidin der Anthranilsäure und einem Nitril der Formel $R^1CN$ hergestellt. Wenn $W^1$ NH bedeutet und $W^2$ für O steht, wird die Verbindung der Formel IX aus einem Anthranilsäurehydrazid und einem Nitril der Formel $R^1CN$ hergestellt. Wenn $W^1$ Sauerstoff und $W^2$ NH bedeutet, wird die Verbindung der Formel IX aus einem Hydrazidin der Anthranilsäure und einem reaktiven Derivat der Säure der Formel $R^1COOH$, z.B. einem Halogenid davon, wie $R^1COCl$ oder $R^1COBr$ oder einem Anhydrid der Formel $(R^1CO)_2O$, hergestellt.

<u>Verfahren b):</u> Das reaktive Derivat der Kohlensäure und die allgemeinen Reaktionsbedingungen sind die selben, wie sie unter Verfahren a) beschrieben worden sind, als reaktives Derivat ist ferner ein Halogencyan, z.B. Chlorcyan oder vorzugsweise Bromcyan, geeignet. Eine geeignete Base wie Triethylamin, Pyridin oder Natriumhydroxid kann zugesetzt werden, um die Halogenwasserstoffsäure, die während der Reaktion gebildet wird, zu neutralisieren. Die Cyclisierung erfolgt insbesondere in situ und kann durch Säure, wie Mineralsäure, z.B. Salzsäure, oder Base, wie ein Trialkylamin, katalysiert werden.

Die Verbindungen der Formel III können nach dem Verfahren von Potts et al., J. Org. Chem. <u>35</u>, 3448 (1970) hergestellt werden. Diese Verbindungen können auch hergestellt werden, indem ein reaktiver Vorläufer der Anthranilsäure, wie Isatosäureanhyrid, mit einem Hydrazidin der Formel $R^1\overset{NH}{\overset{\|}{C}}NHNH_2$ umgesetzt wird, oder indem ein Thioamid der Formel $R^1\overset{S}{\overset{\|}{C}}-NH_2$ mit einem Anthranilsäurehydrazid umgesetzt wird.

<u>Verfahren c):</u> Der Rest eines Kohlensäurederivates Z ist z.B. Isocyanato oder Isothiocyanato; -NHC(=O)O-Niederalkyl oder -NHC(=S)O-Niederalkyl; oder -NHC(=O)N-Diniederalkyl oder -NHC(=S)N-Diniederalkyl oder ein entsprechendes Imidoderivat, wie Cyanimido(-NH-CN).

Die Umsetzung wird vorzugsweise in einem Lösungsmittel durchgeführt, wie einem Ether, z.B. Dioxan oder einem Alkohol, z.B. 2-Methoxyethanol, oder einem flüssigen Amid, z.B. Dimethylacetamid.

Wenn Z Isocyanato oder Isothiocyanato bedeutet, oder -NHC(=O)O-Niederalkyl oder -NHC(=S)O-Niederalkyl, wird die Umsetzung vorteilhaft in Gegenwart einer Base durchgeführt, wie eines tertiären Amins, z.B. Trimethylamin, Triethylamin, und insbesondere Tripropylamin. Die Verbindungen der Formel IV, in denen Z Isocyanato und Isothiocyanato bedeutet, können in die entsprechenden Verbindungen umgewandelt werden, in denen Z -NHC(=O)O-Niederalkyl und -NHC(=S)O-Niederalkyl bedeutet, indem sie mit einem Niederalkanol, wie Ethanol, behandelt werden.

Die Verbindungen der Formel IV, in denen Z für -NHC(=O)O-Niederalkyl oder für -NHC(=S)O-Niederalkyl steht, können auch hergestellt wird, indem ein o-Aminobenzonitril mit Chlorkohlensäureniederalkylester oder Chlorthiokohlensäureniederalkylester umgesetzt wird, z.B. mit Chlorkohlensäureethylester oder Chlorthiokohlensäureethylester.

Die Verbindungen der Formel IV, in denen Z -NHC(=O)N-Diniederalkyl oder -NHC(=S)N-Diniederalkyl bedeutet, können hergestellt werden, indem das geeignete o-Isocyanatobenzonitril oder o-Isothiocyanatobenzonitril mit einem Diniederalkylamin, wie Diethylamin, umgesetzt wird.

Die bevorzugten Lösungmittel, wenn Z Isocyanato oder Isothiocyanato bedeutet, sind Ether, insbesondere Dioxan und ganz besonders Amide, wie 1-Methyl-2-pyrrolidon. Die bevorzugten Lösungsmittel, wenn Z -NHC(=O)O-Niederalkyl oder -NHC(=S)O-Niederalkyl; oder -NHC(=O)N-Diniederalkyl oder -NHC(=S)N-Diniederalkyl bedeutet, sind Alkohole, insbesondere 2-Methoxyethanol und ganz besonders Amide, wie 1-Methyl-2-pyrrolidinon oder Dimethylacetamid. Die Umsetzung wird vorzugsweise bei Temperaturen von 0 bis 250°C vorgenommen, insbesondere zwischen 20 und 150°C.

Für Verbindungen, in denen X für NH steht, werden als Ausgangsverbindungen z.B. o-Cyanimidobenzonitrile benötigt. Solche Verbindungen sind von Wentrup in Tetrahedron 27, 367 (1971) und von Bedford et al. in J. Chem. Soc., 1633 (1959) beschrieben.

Vermutlich handelt es sich bei der Verbindung der Formel X

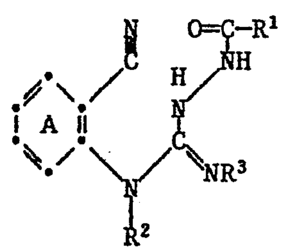

(X)

worin $R^1$, $R^2$, $R^3$ und Ring A wie vorstehend definiert sind, um eine Zwischenstufe in der vorstehend beschriebenen Cyclisierung. Die doppelte Cyclisierung ausgehend von einer Verbindung der Formel X ist integrierter Bestandteil des vorstehend beschriebenen Verfahrens.

Verfahren d): Eine Gruppe, die sich durch ein Oxidationsmittel in die Gruppe -N=C=O überführen lässt, kann jede Gruppe sein, die für Umlagerungsreaktionen wie die nach Hofmann, Curtius oder Lossen von Bedeutung ist, und ist z.B. Carbamoyl, N-Hydroxycarbamoyl oder Azidocarbonyl.

Das Oxidationsmittel kann z.B. Bleitetraacetat oder ein Hypohalogenit sein. Das Hypohalogenit ist vorzugsweise ein Alkalimetallhypohalogenit, wie Natriumhypochlorit oder Natriumhypobromit. Die besagte Gruppe durchläuft vermutlich die erste Stufe z.B. der Hofmann Reaktion (Ber. 14, 2725 (1881)), wobei das Isocyanat gebildet wird, das dann mit dem freien NH des Triazols reagiert.

Verfahren e): Eine Verbindung der Formel VII, worin L Halogen, Niederalkoxy oder Arylniederalkoxy bedeutet, kann hydrolysiert werden. Die Hydrolyse wird vorzugsweise durch Base, z.B. durch Natronlauge vorgenommen.

Die 5-Halogenverbindungen können hergestellt werden, indem eine Verbindung der Formel I, worin X für Sauerstoff steht, mit einem reaktiven Halogenid, wie Phosphorylchlorid, P,P-Dichlorphenylphosphinoxid oder Phosphorpentachlorid, mit oder ohne inertes Lösungsmittel umgesetzt wird.

Die 5-Niederalkoxy oder 5-Arylalkoxyverbindungen können aus den 5-Halogenverbindungen durch Behandeln mit dem geeigneten Alkohol in Anwesenheit von Base hergestellt werden.

Ferner kann ein 5-Halogen, 5-Mercapto oder 5-Alkylmercapto-substituiertes-[1,2,4]triazolo[1,5-c]chinazolin durch Ammoniak oder substituiertes Ammoniak substituiert werden, sodass Verbindungen der Formel I erhalten werden, worin X für $NR^3$ steht. Die Mercaptogruppe kann durch Niederalkyl z.B. Methyl, substituiert werden, indem die 5-Mercaptoverbindung mit z.B. Methylchlorid in Gegenwart einer Base wie Natriumhydrid umgesetzt wird.

Die Verbindung der Formel VII, worin L für -SCN steht, kann mit Ammoniak oder einem Amin $H_2NR^3$ in einem polaren aprotischen Lösungsmittel, vorzugsweise etwa bei Raumtemperatur, umgesetzt werden. Die 5-SCN-Verbindungen können aus den entsprechenden 5-Thionen hergestellt werden, indem ein solches 5-Thion mit Bromcyan in Gegenwart einer Base wie z.B. Natriumhydrid behandelt wird.

Verfahren f): Ein reaktives Derivat einer Carbonsäure der Formel $R^1$-COOH kann ein entsprechendes Säurehalogenid sein, z.B. ein Säurechlorid, ein Ester, wie ein Niederalkylester, ein Iminoether oder -thioether, ein Thioamid oder, bevorzugt, ein Amidin.

Die Umsetzung kann mit oder ohne Lösungsmittel ausgeführt werden, wie in Anwesenheit eines Amides, z.B. von Dimethylacetamid, vorzugsweise unter Erhitzen.

Die Ausgangsmaterialien der Formel VIII können hergestellt werden, indem eine Verbindung der Formel IV, worin Ring A wie vorstehend definiert ist und Z eine der Gruppen -N=C=S, -N=C=O, -NH-CN oder $-N=C=N-R^3$ bedeutet, mit Hydrazin oder einem reaktiven Derivat davon umgesetzt wird, z.B. bei Raumtemperatur in einem inerten Lösungsmittel, wie einem Ether, z.B. Tetrahydrofuran. Dieses Verfahren ist bevorzugt bei der Herstellung von Verbindungen der Formel I, worin X für Sauerstoff und, insbesondere, für Schwefel steht.

Weiterhin kann eine erhaltene Verbindung in eine andere Verbindung der Erfindung übergeführt werden. Z.B. können die 5-Thionverbindungen in die 5-Oxoverbindungen übergeführt werden, indem sie mit einem Hypohalogenitsalz wie Natriumhypochlorit oder Natriumhypobromit behandelt werden. Die 5-Imino- oder substituierte Iminoverbindungen können mit wässriger Säure zu den entsprechenden 5-Oxo-verbindungen hydrolysiert werden. Verbindungen der Formel I, worin $R^2$ für Wasserstoff steht und X Sauerstoff bedeutet, können in Verbindungen übergeführt werden, worin $R^2$ Niederalkyl bedeutet, z.B. durch

Umsetzen mit einem Alkylhalogenid in Anwesenheit einer Base, wie Natriumalkoxid, in einem inerten Lösungsmittel, wie Dimethylsulfoxid.

In den vorstehend beschriebenen Herstellungsverfahren für die Verbindungen der Erfindung werden die Umsetzungen unter Standardbedingungen durchgeführt. Beispielsweise können die Ansätze auf geeignete Temperaturen abgekühlt oder erhitzt werden, geeignete Lösungsmittel und Katalysatoren können zugesetzt werden, und die Reaktion kann unter Inertgasatmosphäre durchgeführt werden.

Die beanspruchten Salze und freien Verbindungen sind ineinander überführbar. Beispielsweise können Säureadditionssalze in freie Verbindungen übergeführt werden, indem sie mit einer geeigneten Base behandelt werden, und freie Verbindungen können in die entsprechenden Säureadditionssalze übergeführt werden, indem sie mit der entsprechenden Säure behandelt werden.

Die Ausgangsmaterialien für die Herstellung der Verbindungen der Erfindung sind entweder bekannt oder können auf an sich bekannte Weise hergestellt werden.

Die Verbindungen der Formel I lassen sich zur Herstellung von pharmazeutischen Zusammensetzungen verwenden, die eine wirksame Menge der Triazolochinazolinverbindungen der Formel I oder ein Salz davon enthalten, in Kombination mit üblichen Zusatzstoffen oder Trägern, die für die enterale, parenterale wie orale, bronchiale, rektale oder intravenöse Verabreichung geeignet sind. Bevorzugt sind Tabletten, Dragées und Gelatinekapseln, die die aktive Komponente gemeinsam mit a) Verdünnungsmitteln, z.B. Lactose, Dextrose, Sucrose, Mannit, Sorbit, Cellulose, Calciumphosphat und/oder Glycin, b) Gleitmitteln, z.B. Silica, Talk, Stearinsäure, deren Magnesium- oder Calciumsalze und/oder Polyethylenglycol, für Tabletten auch c) Bindemitteln, z.B. Magnesiumaluminiumsilikat, Stärke, Gelatine, Traganth, Methylcellulose, Natriumcarboxymethylcellulose und/oder Polyvinylpyrrolidon, wenn erwünscht d) Sprengmitteln, z.B. Stärken,

Agar, Alginsäure oder Natriumalginat, oder schäumende Mischungen, und/oder e) Absorbentien, Farbstoffen, Geschmacks- und Süssstoffen enthalten. Dragées oder Tablettenkerne können mit geeigneten Ueberzügen versehen sein, die magensaftresistent sein können. Ueberzugslösungen sind z.B. konzentrierte wässrige Zuckerlösungen, die Gummi arabicum, Polyvinylpyrrolidon, Polyethylenglykol, Talk und/oder Titandioxid enthalten können. Widerstandsfähige Ueberzüge werden mit Lacklösungen in organischen Lösungsmitteln erhalten, wie Schellack, Acetylcellulosephthalat oder Hydroxypropylmethylcellulosephthalat in Ethanol und dergleichen. Farbstoffe oder Pigmente können zur Identifizierung des Namens und der Dosis zugesetzt werden. Kapseln werden entweder aus Hartgelatine hergestellt, oder es sind weiche geschlossene Kapseln als Gelatine und einem Weichmacher, z.B. Glycerin oder Sorbit. Die Hartkapseln enthalten entweder unverpresste Pulvergemische, z.B. solche wie unter a) und b) erwähnt, oder Granulate ähnlich denen, die für Tabletten verwendet werden. In den Weichkapseln sind die aktiven Wirkstoffe vorzugsweise in geeigneten Flüssigkeiten gelöst oder suspendiert, wie in Fettölen, Paraffinen oder Polyethylenglykolen.

Suppositorien sind vorzugsweise feste Fettemulsionen oder Suspensionen, die den Wirkstoff z.B. in natürlichen oder synthetischen Triglyceriden, Paraffinen, Wachsen und/oder Polyethylenglykolen enthalten.

Zusammensetzungen zur parenteralen Verabreichung sind vorzugsweise wässrige Lösungen oder Suspensionen dieser Verbindungen, aber auch ölige Lösungen oder Suspensionen davon, z.B. in natürlichen oder synthetischen Fettölen, wie Sesamöl oder Oelsäureethylester, in geeigneten Ampullen.

Bronchialzusammensetzungen sind vorzugsweise Aerosolsprays und können aus einem Dispenser verabreicht werden, wie er in den US Patenten 4,292,966, 4,174,712 und 4,137,914 beschrieben ist. Der

aktive Bestandteil wird mit einem Treibmittel vermischt wie einem Kohlenwasserstoff, einem Chlorfluorkohlenwasserstoffgemisch oder Kohlendioxid.

Diese Zusammensetzungen können sterilisiert sein und/oder Zusatzstoffe enthalten, wie Konservierungs-, Stabilisierungs-, Befeuchtungs- oder Emulgierungsmittel, Lösungsvermittler, Salze zur Regulierung des osmotischen Drucks und/oder Puffer. Sie können auch andere therapeutisch wertvolle Substanzen enthalten, und werden durch herkömmliche Techniken wie Mischen, Granulieren oder entsprechende Ueberzugsmethoden hergestellt. Sie können zwischen 10 und 95 %, vorzugsweise zwischen 20 und 70 % der aktiven Verbindung enthalten. Individuelle Einheitsdosen für einen Säuger von etwa 50 bis 70 kg Gewicht können vorzugsweise zwischen 10 und 200 mg insbesondere zwischen 20 und 100 mg besagter aktiver Wirkstoffe enthalten.

Nachdem die Erfindung wie vorstehend in allgemeiner Form beschrieben wurde, werden in der Folge zum detaillierteren Verständnis spezifische Beispiele angeführt, die nur zur Illustrierung dienen und den Gegenstand der Erfindung in keiner Weise, etwa auf den Umfang der Beispiele, beschränken sollen. Temperaturen werden in Celsius Graden angegeben, Verdampfungen unter vermindertem Druck, wenn nicht anderweitig angegeben, werden bei 1 bis 13 kpa durchgeführt.

<u>Beispiel 1:</u> 7 g 3-(2-Carbamoylphenyl)-5-(4-chlorphenyl)-1H-1,2,4-triazol werden bei 50° in 180 ml trockenem Dimethylformamid gelöst. 10,4 g Bleitetraacetat werden unter Rühren zugegeben, und der Ansatz wird 10 weitere Minuten unter Stickstoff gerührt. Der Ansatz wird in ein Gemisch aus 500 ml zerstossenem Eis und 50 ml konzentrierter Salzsäure gegeben. Der weisse Festkörper, der sich abscheidet, wird isoliert, intensiv mit kaltem Wasser gewaschen und aus 2-Methoxyethanol umkristallisiert. Man erhält 2-(4-Chlorphenyl)[1,2,4]-triazolo[1,5-c]chinazolin-5(6H)on, Fp. 315-318°.

Das Ausgangsmaterial wird wie folgt hergestellt: Ein Gemisch von 61 g 1-Hydrazinophthalazinhydrochlorid, 43,4 g p-Chlorbenzaldehyd und 1200 ml Methanol wird 18 Stunden unter Stickstoff unter Rückfluss erhitzt. Das Gemisch wird unter Wasserstrahlvakuum eingeengt, einige Minuten gerührt und filtriert. Der erhaltene gelbe Feststoff wird mit Ether gewaschen (3 x 300 ml), das p-Chlorphenylhydrazon wird isoliert und unmittelbar in die folgende Reaktion eingesetzt:

120 g dieses p-Chlorphenylhydrazons werden in einem Gemisch aus 2300 ml Eisessig und 160,1 g wasserfreiem Natriumacetat einige Minuten bei Raumtemperatur unter Stickstoff gerührt. 26,2 ml Brom in 150 ml Eisessig werden zugetropft, danach wird der Ansatz 12 Stunden bei 90° gerührt, abgekühlt und filtriert. Das Filtrat wird unter vermindertem Druck zur Trockne eingeengt. Der erhaltene Feststoff wird in 800 ml Wasser eingerührt, mit Natronlauge neutralisiert und 18 Stunden gerührt. Das Produkt, 3-(4-Chlorphenyl)-1,2,4-triazolo-[3,4-a]phthalazin, Fp. 222-225°, wird ohne Reinigung weiter verwendet. Die reine Verbindung schmilzt nach Umkristallisieren aus Ethanol bei 224-225°.

Zu einem Gemisch von 12,7 g Natriummethylat und 1500 ml Ethanol werden 66 g des beschriebenen Triazolophthalazins gegeben. Der Ansatz wird 6 Tage unter Stickstoff unter Rückfluss erhitzt. Das Gemisch wird unter vermindertem Druck auf 100 ml eingeengt, mit 1000 ml Wasser verdünnt, 2 Stunden gerührt, filtriert und unter Kühlen mit Salzsäure auf pH 5 eingestellt. Das feste 3-(4-Chlorphenyl)-5-(2-cyanphenyl)-1H-1,2,4-triazol wird isoliert, mit Wasser gewaschen und an der Luft getrocknet. Das Material ist für den nächsten Schritt ausreichend sauber, kann aber auch durch Umkristallisieren aus einem Gemisch von Ethanol und Isopropanol weiter gereinigt werden, man erhält so einen weissen Festkörper, Fp. 236-238°.

5 g des beschriebenen rohen Nitrils werden in 20 ml 85%iger Schwefelsäure gelöst, 90 Minuten bei 80° gerührt und in 400 ml Eiswasser gegeben. Der Niederschlag wird isoliert, mit Wasser gewaschen, auf

dem Filter trockengepresst und mit Ether gewaschen. Das Produkt, 3-(2-Carbamoylphenyl)-5-(4-chlorphenyl)-1H-1,2,4-triazol wird aus 2-Methoxyethanol umkristallisiert, man erhält so das reine Amid, Fp. 234-237°.

Beispiel 2: Nach dem Verfahren des Beispiels 1 wird aus 3-(2-Carbamoylphenyl)-5-phenyl-1H-1,2,4-triazol das 2-Phenyl[1,2,4]triazolo-[1,5-c]chinazolin-5(6H)on, Fp. 311-313° nach Umkristallisieren aus Ethanol, erhalten. Die Umsetzung erfolgt während einer Stunde bei 80°. Die Carbamoyl-Verbindung, Fp. 222-225°, wird nach Druey, Ringier, Helv. Chim. Acta 34, 195-210 (1951) ausgehend von 3-Phenyl-1,2,4-triazolo[3,4-a]phthalazin über das Nitril hergestellt, wie in Beispiel 1 beschrieben.

Beispiel 3: Zu einer Lösung von 20,5 g 3-(2-Carbamoylphenyl)-5-(3-pyridyl)-1H-1,2,4-triazol in 600 ml trockenem Dimethylformamid werden 9,4 g Eisessig gegeben, unter Rühren bei 40° werden 35 g Bleitetraacetat zugesetzt. Die dunkelrote Mischung wird unter Stickstoff 66 Stunden bei 85° gerührt, in 2000 ml Eiswasser gegeben und gekühlt. Nach 20 Stunden wird der abgeschiedene Feststoff isoliert, in heissem Ethanol gelöst und über Diatomeenerde filtriert. Das ethanolische Filtrat wird mit 4,7 g p-Toluolsulfonsäurehydrat behandelt. Zugabe von trockenem Ether ergibt das Tosylat von 2-(3-Pyridyl)[1,2,4]triazolo[1,5-c]chinazolin-5(6H)on, Fp. 268-271°. Einengen der wässrigen Mutterlauge und kontinuierliche Extraktion mit Ether ergibt eine weitere Menge weniger reines Material.

Das Ausgangsamid, Fp. 248-250° nach Umkristallisieren aus 2-Methoxyethanol, wird aus 3-(3-Pyridyl)-1,2,4-triazolo[3,4-a]phthalazin [Haase, Biniecki, Chem. Abstracts 61, 3103b (1964)] über das Nitril hergestellt, wie in Beispiel 1 beschrieben, ausser dass das Nitril und Amid bei neutralem pH isoliert werden.

<u>Beispiel 4:</u> Zu einem Gemisch von 6 g 2-(4-Chlorphenyl)[1,2,4]-triazolo[1,5-c]chinazolin-5(6H)on und 150 ml trockenem Dimethylsulfoxid werden 1,3 g Natriummethylat gegeben. Die Mischung wird eine Stunde unter Stickstoff auf 85° erhitzt, und 1,3 ml Methyliodid in 10 ml Dimethylsulfoxid werden zugesetzt. Es bildet sich schnell ein weisser Feststoff. Der Ansatz wird drei weitere Stunden erhitzt, abgekühlt und in 800 ml Eiswasser gegeben. Der ausgefallene weisse Feststoff wird mit Wasser gewaschen, an der Luft getrocknet und zweimal aus Dimethylacetamid umkristallisiert. Man erhält reines 2-(4-Chlorphenyl)-6-methyl[1,2,4]triazolo[1,5-c]chinazolin-5(6H)on, Fp. 323-325°.

<u>Beispiel 5:</u> Nach der selben Methode wie in Beispiel 4 wird 2-Phenyl-[1,2,4]triazolo[1,5-c]chinazolin-5(6H)on quantitativ in die rohe 6-Methyl-Verbindung übergeführt und dann durch Umkristallisieren aus Tetrahydrofuran gereinigt, Fp. 231-234°.

<u>Beispiel 6:</u> Eine Mischung von 10,4 g 2-Phenyl-[1,2,4]triazolo-[1,5-c]chinazolin-5(6H)on und 150 ml trockenem Dimethylformamid wird mit 1,8 g einer 57%igen Dispersion von Natriumhydrid in Mineralöl behandelt, unter Stickstoff auf 100° erhitzt und 10 Minuten später während 20 Minuten tropfenweise mit 7,7 g trans-Cinnamylbromid in 75 ml Dimethylformamid versetzt. Das Gemisch wird unter Stickstoff vier Stunden bei 65° gerührt, gekühlt und in Eiswasser gegeben. Der rohe Niederschlag wird aus Dimethylacetamid und dann aus Dimethylsulfoxid umkristallisiert. Man erhält so das reine 6-trans-Cinnamylderivat, Fp. 167-169°.

<u>Beispiel 7:</u> Ein Gemisch von 13 g 2-Phenyl[1,2,4]triazolo[1,5-c]-chinazolin-5(6H)on und 100 ml trockenem Dimethylformamid wird mit 2,05 g einer 57%igen Dispersion von Natriumhydrid in Mineralöl behandelt und zwei Stunden unter Stickstoff gerührt. Zur klaren Lösung werden 0,2 g Kaliumiodid, danach 2,05 g der Dispersion von Natriumhydrid in Oel und darauf 8,3 g β-3-Picolylchlorid ganz allmählich zugegeben, wobei sich die Temperatur auf 50° erhöht. Der Ansatz wird unter Stickstoff 20 Stunden bei 50° gerührt und auf

600 g zerstossenes Eis gegeben. Der weisse Feststoff wird isoliert mit Wasser gewaschen und in 400 ml siedendem Ethanol aufgenommen. Nach Filtrieren wird das Filtrat mit Methansulfonsäure auf pH 2 eingestellt. Das rohe Salz wird aus Ethanol umkristallisiert und in 100 ml eines Gemischs aus Ether und 0,5 N Natronlauge suspendiert. Die freie Base wird isoliert und durch Umkristallisieren aus Isopropanol gereinigt. Man erhält das reine 6-(3-Picolyl)-Derivat, Fp. 202-204°.

Beispiel 8: Eine Lösung von 7,3 g 2-(3-Pyridyl)[1,2,4]triazolo-[1,5-c]chinazolin-5(6H)on-p-toluolsulfonat in 60 ml trockenem Dimethylsulfoxid wird mit 1,4 g einer 57%igen Dispersion von Natriumhydrid in Mineralöl behandelt. Der Ansatz wird unter Stickstoff eine Stunde auf 60° erhitzt und dann mit 2,4 g Methyliodid behandelt. Nach 24-stündigem Rühren unter Stickstoff bei 60° wird die Mischung in 500 ml Eiswasser gegeben, der Niederschlag isoliert, mit Wasser gewaschen und an der Luft getrocknet. Er wird in 400 ml Methanol suspendiert, mit p-Toluolsulfonsäure auf pH 4 eingestellt, und das Salz wird durch Zugabe von Ether ausgefällt. Das rohe Salz wird in 2N Natronlauge suspendiert, isoliert, mit wässriger Natriumthiosulfatlösung gewaschen, danach mit Wasser, Ethanol und erneut in das Tosylat übergeführt. Das Salz wird aus Ethanol umkristallisiert. Man erhält das reine 6-Methyl-Derivat als Tosylat, Fp. 217-219°.

Beispiel 9: Eine Mischung von 15 g 1-(2-Aminobenzoyl)-2-(isonicotinimidoyl)hydrazin und 300 g Ethylcarbamat wird viereinhalb Stunden unter Stickstoff bei einer Aussentemperatur von 210° am Wasserabscheider gerührt. Dabei werden etwa 90 ml Flüssigkeit erhalten. Das verbleibende Material, das beim Abkühlen fest wird, wird bei 60° eine Stunde in 500 ml Wasser gerührt, isoliert, kräftig mit 500 ml Essigester gerührt und erneut isoliert. Das Rohprodukt wird dann in Wasser gelöst, mit Natriumhydroxid auf pH 13 eingestellt, filtriert und mit Säure neutralisiert. Das ausgefallene Produkt wird in 200 ml 2-Methoxyethanol suspendiert, mit p-Toluolsulfonsäure angesäuert und mit Ether behandelt. Das ausgefallene Salz wird gereinigt, indem es in die freie Base übergeführt, in das Tosylat rücküberführt und aus

Methanol umkristallisiert wird. Man erhält so reines 2-(4-Pyridyl)-[1,2,4]triazolo[1,5-c]chinazolin-5(6H)on-p-toluolsulfonat, Fp. 271-274°.

Das Ausgangsmaterial wird wie folgt hergestellt: 16 g 4-Pyridylhydrazidin werden mit 16,3 g Isatosäureanhydrid gemischt, 200 ml trockenes Pyridin werden zugegeben, und der Ansatz wird über Nacht bei Raumtemperatur unter Stickstoff gerührt. Der Niederschlag wird isoliert, mit 50 ml Tetrahydrofuran und 500 ml trockenem Ether gewaschen und an der Luft getrocknet. Man erhält das Produkt vom Fp. 231-235°. Versetzen der Mutterlauge mit 1000 ml Petrolether ergibt eine weitere Menge vom Fp. 221-224°.

Beispiel 10: Nach dem Verfahren des Beispiels 9 wird 3-Pyridylhydrazidin mit 5-Chlorisatosäureanhydrid behandelt. Das Produkt, Fp. 189-193°, wird mit Ethylcarbamat kondensiert. Man erhält so 9-Chlor-2-(3-pyridyl)[1,2,4]triazolo[1,5-c]chinazolin-5(6H)on als p-Toluolsulfonat, Fp. 320-322°.

Beispiel 11: Ein Gemisch von 6 g 3-(2-Amino-3,5-dichlorphenyl)-5-phenyl-1H-1,2,4-triazol und 50 g Ethylcarbamat wird 6 Stunden unter Stickstoff auf 195° erhitzt, danach auf 80° abgekühlt, in 1000 ml Wasser gegeben und 16 Stunden gerührt. Das Produkt wird isoliert, mit Wasser gewaschen, danach mit 300 ml Methanol und an der Luft getrocknet. Das Rohmaterial wird aus 2-Methoxyethanol/Wasser (12:1) umkristallisiert. Man erhält reines 7,9-Dichlor-2-phenyl[1,2,4]-triazolo[1,5-c]chinazolin-5(6H)on, Fp. 309-311°.

Das als Ausgangsmaterial verwendete Triazol wird wie folgt hergestellt: 25 g Benzamidinhydrochlorid werden in 100 ml Methanol gelöst und mit 13 g 50%iger Natronlauge behandelt. Das Salz wird abfiltriert, die Lösung wird zu 26 g 2-Amino-3,5-dichlorbenzoesäurehydrazid in 300 ml Toluol gegeben. Der Ansatz wird unter Stickstoff 60 Stunden in einer Apparatur mit Wasserabscheider unter Rückfluss erhitzt. Die abgeschiedene Flüssigkeit wird entfernt, frisches Toluol wird innerhalb dieser Zeit zugesetzt. Die Mischung wird unter

vermindertem Druck auf 75 ml eingeengt und mit 300 ml Petrolether behandelt. Das weisse Produkt wird isoliert und aus Methanol umkristallisiert. Man erhält das reine Produkt, Fp. 235-238°.

Beispiel 12: Nach dem Verfahren des Beispiels 11 wird 3-(2-Amino-5-chlorphenyl)-3-(2-pyridyl)-1H-1,2,4-triazol in 9-Chlor-2-(2-pyridyl)[1,2,4]triazolo[1,5-c]chinazolin-5(6H)on, Fp. >340°, übergeführt. Das als Ausgangsmaterial verwendete Triazol wird wie folgt hergestellt: Eine Mischung von 12 g 5-Chlorisatosäureanhydrid, 8,3 g 2-Pyridylhydrazidin und 150 ml Pyridin wird 20 Stunden unter Stickstoff gerührt, 200 ml Ether werden zugesetzt, und das Produkt, Fp. 230-232°, wird isoliert. Diese Zwischenstufe, 1-(2-Amino-5-chlorbenzoyl)-2-picolinimidoylhydrazin, wird in 150 ml eines Gemisches aus Diphenylether/Biphenyl (3:1) gegeben, 5 Stunden unter Stickstoff bei 180° gerührt, abgekühlt und mit 200 ml Hexan behandelt. Man isoliert das Produkt und erhält so, nach Umkristallisieren aus Ethanol, besagtes Triazol, Fp. 207-209°.

Beispiel 13: Eine Suspension von 7 g 3-(2-Amino-5-chlorphenyl)-5-phenyl-1H-1,2,4-triazol in 100 ml trockenem Dioxan wird mit 5,1 g Chlorkohlensäuretrichlormethylester behandelt und unter Stickstoff 90 Minuten bei Raumtemperatur gerührt. 2,62 g Triethylamin werden zugegeben, und es wird weitere 18 Stunden gerührt. Der Ansatz wird dann eine Stunde unter Rückfluss gekocht und abgekühlt, der Feststoff wird isoliert, mit Ether, danach mit Wasser gewaschen und dann in Wasser suspendiert und 90 Minuten kräftig gerührt. Das Produkt Fp. >340° ist reines 9-Chlor-2-phenyl[1,2,4]triazolo[1,5-c]-chinazolin-5(6H)on.

Das als Ausgangsmaterial verwendete Triazol wird wie folgt hergestellt: Wie in Beispiel 11 beschrieben wird aus 4,64 g des Hydrochlorids in 110 ml Ethanol die freie Benzamidinbase hergestellt. Sie wird in einer Apparatur mit Wasserabscheider zu einer Lösung von 5 g 2-Amino-5-chlorbenzoesäurehydrazid in 200 ml Chlorbenzol/Ethanol (9:1) gegeben. Der Ansatz wird unter Stickstoff vier Stunden unter Rückfluss gekocht, dabei scheiden sich 130 ml Flüssigkeit ab, und

weiteres Chlorbenzol wird zugesetzt, um die Flüssigkeitsmenge etwa konstant zu halten. Das Gemisch wird abgekühlt, 200 ml Ether werden zugesetzt, das Produkt wird isoliert, mit Ether gewaschen und an der Luft getrocknet. Das Triazol, Fp. 254-255°, ist analytisch rein.

Beispiele 14-21: Auf analoge Weise wie in Beispiel 13 beschrieben werden Verbindungen der Formel I, worin $R^2$ Wasserstoff und X Sauerstoff bedeuten, aus den entsprechenden 3-(2-Aminophenyl)-5-substituierten-1H-1,2,4-triazolen hergestellt, die sich ihrerseits aus dem entsprechenden Amidin und 2-Amino-5-chlorbenzoesäurehydrazid herstellen lassen:

| Nummer des Beispiels | Substituent von Ring A | $R^1$ | Fp. Produkt | Fp. des verwendeten Triazols |
|---|---|---|---|---|
| 14 | 9-Cl | 4-Methylphenyl | >320° | >300° |
| 15 | 9-Cl | 4-Fluorphenyl | >320° | 254-257° |
| 16 | 9-Cl | 4-Methoxyphenyl | >320° | 217-219° |
| 17 | 9-Cl | 3,4,5-Trimethoxyphenyl | >290° | 243-248° |
| 18 | 9-Cl | 2-Furyl | >340° | 236-237° |
| 19 | 9-Cl | 2-Thienyl | >330 | 250-251° |
| 20 | 9-Cl | 3-Fluorphenyl | >320° | 260-263° |
| 21 | 9-Cl | 4-Trifluormethylphenyl | 334-336° | 215-217° |

Beispiel 22: Analog zum Beispiel 13 wird 3-(2-Amino-5-chlorphenyl)-5-(2-fluorphenyl)-1H-1,2,4-triazol in das 9-Chlor-2-(2-fluorphenyl)-[1,2,4]triazolo[1,5-c]chinazolin-5(6H)on, Fp. >360°, übergeführt.

Das Triazol wird wie folgt hergestellt: Ein Gemisch von 6,0 g 2-Fluorbenzamid und 8,7 g 2,4-Bis-(4-methoxyphenyl)-2,4-dithioxo-1,3-dithia-2,4-diphosphetan (Lawessons Reagens) in 100 ml Toluol wird drei Stunden unter Rückfluss gekocht und gerührt, unter vermindertem Druck zur Trockne eingeengt und mit trockenem Ether

verrieben. Der so gewonnene Feststoff wird erneut mit Ether gewaschen. Die vereinigten Etherphasen werden über Silicagel filtriert und eingeengt. Man erhält so das feste Thioamid, das ohne weitere Reinigung verwendet wird. Das Thioamid wird zusammen mit 7,9 g 2-Amino-5-chlorbenzoesäurehydrazid in 100 ml eines Gemisches von Diphenylether/Biphenyl (3:1) gegeben und 20 Stunden unter Stickstoff bei 180° gerührt. Der abgekühlte Ansatz wird mit 200 ml Hexan verdünnt und filtriert. Der Niederschlag wird durch Hochdruckflüssigkeitschromatographie in 2-(2-Amino-5-chlorphenyl)-5-(2-fluorphenyl)-1,3,4-oxadiazol, Fp. 168-170°, und das gewünschte Triazol Fp. 211-213°, aufgetrennt.

Beispiel 23: 1,36 g Benzoylhydrazin in 50 ml trockenem Dioxan werden mit 1,44 g o-Isocyanatobenzonitril in 20 ml Dioxan versetzt und eine Stunde bei 80° unter Stickstoff gehalten. Nach dem Abkühlen werden 1,87 g Feststoff isoliert. Eindampfen der Mutterlauge und Behandeln mit Ether ergibt eine weitere Menge Feststoff. Das vereinigte Material, Fp. >295°, wird direkt in die nächste Stufe eingesetzt. Es wird in eine Mischung aus 30 ml konzentrierter Ammoniumhydroxidlösung und 120 ml Ethanol gegeben und 2 Stunden unter Stickstoff unter Rückfluss erhitzt. Die Lösung wird unter vermindertem Druck teilweise eingedampft, so dass das Ethanol abgezogen wird, und gekühlt. Der Niederschlag wird isoliert und mit Wasser gewaschen. Das Produkt, Fp. 310-313° ist identisch mit dem 2-Phenyl[1,2,4]-triazolo[1,5-c]chinazolin-5(6H)on, das in Beispiel 2 beschrieben ist.

Beispiel 24: Auf analoge Weise wie in Beispiel 23 wird die Zwischenstufe mit dem Fp. 300°, hergestellt aus 2-Isocyanatobenzonitril und 2-Fluorbenzoylhydrazin, in 2-(2-Fluorphenyl)[1,2,4]triazolo[1,5-c]-chinazolin-5-(6H)on, Fp. 326-329°, übergeführt.

Beispiel 25: Die Zwischenstufe, die aus 6,1 g 2-Isocyanato-5-chlorbenzonitril und 7,0 g 4-Hydroxybenzoylhydrazin wie in Beispiel 23 beschrieben hergestellt ist, wird zu einer Mischung von 31 ml Triethylamin, 25 Tropfen Methansulfonsäure und 300 ml Ethanol

gegeben und unter Stickstoff zwei Stunden unter Rückfluss gerührt. Der Ansatz wird unter vermindertem Druck zur Trockne eingeengt, in Ethanol aufgenommen und mit Wasser behandelt. Der Feststoff wird isoliert, mit Wasser gewaschen und im Hochvakuum getrocknet. Man erhält reines 9-Chlor-2-(4-hydroxyphenyl)[1,2,4]triazolo[1,5-c]-chinazolin-5(6H)on, Fp. >350°.

Beispiel 26: Die Zwischenstufe aus 2-Tetrahydrofuroylhydrazin und 5-Chlor-2-isocyanatobenzonitril, hergestellt analog zur in Beispiel 23 geschilderten Methode, wird, wie in Beispiel 25 beschrieben, in 9-Chlor-2-(2-tetrahydrofuryl)[1,2,4]triazolo[1,5-c]-chinazolin-5(6H) on, Fp. 239-243°, übergeführt.

Beispiel 27: Die Zwischenstufe aus N-Methylpipecolinsäurehydrazid und 5-Chlor-2-isocyanatobenzonitril, hergestellt analog zur in Beispiel 23 geschilderten Methode, wird, wie in Beispiel 25 beschrieben, in 9-Chlor-2-[2-(1-methylpiperidyl)]-[1,2,4]triazolo-[1,5-c]chinazolin-5(6H)on übergeführt und als Fumarat mit dem Fp. 274-276° gereinigt.

Beispiel 28: Eine Suspension von 3,8 g 3-(2-Amino-5-chlorphenyl)-5-(2-pyridyl)-1H-1,2,4-triazol, Vorläufer für Beispiel 12, in 100 ml Methanol wird mit 1,5 g Bromcyan versetzt und 18 Stunden unter Stickstoff unter Rückfluss gerührt. 1,43 g Triethylamin werden zugegeben, es wird eine weitere Stunde unter Rückfluss gerührt. Nach Filtrieren wird der Feststoff mit Methanol und Ether gewaschen, er schmilzt oberhalb von 330°. Er wird in 100 ml Methanol suspendiert, eine äquimolare Menge Methansulfonsäure wird zugegeben, man lässt über Nacht rühren. Das Produkt wird isoliert, mit Methanol gewaschen und im Vakuum getrocknet. Man erhält reines 9-Chlor-5-imino-2-(2-pyridyl)-5,6-dihydro[1,2,4]triazolo[1,5-c]chinazolinmethansulfonat, Fp. 248-250°.

Beispiele 29-36: Auf analoge Weise wie in Beispiel 28 werden die folgenden Verbindungen der Formel I, in denen $R^2$ Wasserstoff bedeutet und X für NH steht, aus den entsprechenden Triazolen hergestellt. In einigen Beispielen wird die freie Base isoliert, in anderen ein anderes Salz als das Methansulfonat (Mesylat).

| Nummer des Beispiels | Substituent von Ring A | $R^1$ | Art des Salzes | Fp. des Produkt |
|---|---|---|---|---|
| 29 | 9-Cl | 4-Methylphenyl | Mesylat | 312-315° |
| 30 | 9-Cl | 4-Fluorphenyl | Tosylat | 310-312° |
| 31 | 9-Cl | 3,4,5-Trimethoxyphenyl | Mesylat | 278-282° |
| 32 | 9-Cl | Phenyl | -- | 284-286° |
| 33 | 9-Cl | 2-Furyl | Mesylat | 275-280° |
| 34 | 9-Cl | 3-Fluorphenyl | -- | >320° |
| 35 | 9-Cl | 2-Thienyl | Mesylat | 247-252° |
| 36 | 9-Cl | 4-Trifluormethylphenyl | Mesylat | 286-290° |

Beispiel 37: 1,41 g 2-(2-Furyl)-5-methylthio[1,2,4]triazolo[1,5-c]-chinazolin werden mit 200 ml Ethanol und 100 ml 70 % wässrigem Ethylamin in einem Autoklaven aus rostfreiem Stahl bei einer Aussentemperatur von 150° erhitzt. Ein Druck von 700 kPa wird erreicht, der Ansatz wird 16 Stunden unter diesen Bedingungen gehalten. Nach dem Abkühlen wird das Material unter vermindertem Druck zur Trockne eingeengt. Der erhaltene Feststoff wird aus Methanol umkristallisiert. Man erhält reines 5-Ethylimino-2-(2-furyl)-5,6-dihydro[1,2,4]triazolo[1,5-c]chinazolin, Fp. 133-135°.

Die als Ausgangsmaterial verwendete 5-Methylthio-Verbindung wird wie folgt hergestellt: Ein Gemisch von 15,8 g 2-Furancarbonsäurehydrazid, 20 g 2-Cyanphenylisothiocyanat und 600 ml trockenem Dioxan wird eine Stunde unter Stickstoff unter Rückfluss gerührt. Der Ansatz wird unter vermindertem Druck zur Trockne eingeengt, der Rückstand mit Methanol verrieben und der Feststoff isoliert. 32,6 g dieser Zwischenstufe, Fp. >300°, werden in einem Gemisch aus 230 ml

Triethylamin, 1800 ml Ethanol und 7 ml Methansulfonsäure suspendiert, zwei Stunden unter Stickstoff unter Rückfluss gerührt, unter vermindertem Druck zu einem dicken Oel konzentriert, in 300 ml Ethanol aufgenommen und gerührt, bis vollständige Kristallisation erfolgt ist. Der Feststoff, rohes 2-(2-Furyl)-5-mercapto[1,2,4]-triazolo[1,5-c]chinazolin, Fp. >300°, wird direkt in die nächste Stufe eingesetzt.

2,01 g dieser Mercapto-Verbindung werden zu einer Lösung von 0,83 g Natriummethylat in 20 ml Methanol gegeben, der Ansatz wird einige Minuten gerührt und dann mit 0,47 ml Methyliodid versetzt. Eine Lösung bildet sich, und nach einstündigem Erhitzen bei einer Badtemperatur von 80° hat sich ein dichter Niederschlag gebildet, der weiter mit Methanol verdünnt wird, abgekühlt und an der Luft getrocknet. Der Feststoff, Fp. 188-190°, wird isoliert, mit Methanol gewaschen und an der Luft getrocknet.

Beispiel 38: In analoger Weise wie bei Beispiel 37 beschrieben wird 2-(2-Furyl)-5-methylthio[1,2,4]triazolo[1,5-c]chinazolin mit 40 % wässrigem Methylamin in Ethanol in 2-(2-Furyl)-5-methylimino-5,6-dihydro[1,2,4]triazolo[1,5-c]chinazolin, Fp. nach Umkristallisieren aus Methanol 193-195°, übergeführt.

Beispiel 39: Wie bei Beispiel 37 wird 2-(2-Furyl)-5-methylthio-[1,2,4]triazolo[1,5-c]chinazolin mit Ammoniumhydroxidlösung, gesättigt mit Ammoniak bei 0°, 6 Stunden bei 150° und einem Druck von 1,5 MPa in einem rostfreien Stahlautoklaven umgesetzt. Man erhält 2-(2-Furyl)-5-imino-5,6-dihydro[1,2,4]triazolo[1,5-c]-chinazolin, Fp. 282-285° nach Umkristallisieren aus Methanol.

Beispiel 40: Ein Gemisch von 4,8 g 3-(2-Methylaminophenyl)-5-(2-furyl)-1H-1,2,4-triazol, 2,17 g Bromcyan und 150 ml Methanol wird 18 Stunden unter Stickstoff unter Rückfluss erhitzt. Das Gemisch wird heiss filtriert, um 600 mg 2-(2-Furyl)-6-methyl[1,2,4]triazolo[1,5-c]chinazolin-5(6H)on, Fp. 238,5-241,5° nach Umkristallisieren aus 2-Methoxyethanol, zu entfernen. Nach Abkühlen wird ein

weisser Feststoff erhalten, der in Methanol aufgenommen und mit 2,8 ml Triethylamin behandelt wird, sodann zwei Stunden unter Rückfluss erhitzt, mit kaltem Wasser verdünnt und durch Filtrieren isoliert wird. Das feste Material wird aus einem 2-Methoxyethanol/Methanol-Gemisch umkristallisiert und dann in Methanol unter Zugabe von Isopropanol unter Kühlen in das Methansulfonatsalz übergeführt. Das Salz wird dann in 300 ml Wasser aufgenommen, das einige Tropfen Methansulfonsäure enthält, zur Reinigung filtriert, unter vermindertem Druck auf ein kleines Volumen eingeengt und durch Zugabe von Isopropanol gefällt. Das Produkt wird 18 Stunden im Hochvakuum bei 100° getrocknet. Man erhält reines 2-(2-Furyl)-5-imino-6-methyl-5,6-dihydro[1,2,4]triazolo[1,5-c]chinazolinmethansulfonat, Fp. 259-263°.

Das Ausgangsmaterial wird wie folgt hergestellt: 14,5 g 2-(Methylamino)benzoesäurehydrazid [Hetter et al., J. prakt. Chem. 111, 36-53 (1925)] werden mit 2-Furanamidin, das wie in Beispiel 13 beschrieben aus 12,9 g des Hydrochlorides hergestellt ist, behandelt; man erhält so das gewünschte Triazol, Fp. 179-183°.

Beispiel 41: In einem Autoklaven aus rostfreiem Stahl werden 50 ml konzentrierte wässrige Ammoniumhydroxidlösung bei 0° mit Ammoniakgas gesättigt, und 1,22 g 5,9-Dichlor-2-(2-tetrahydrofuryl)[1,2,4]-triazolo[1,5-c]chinazolin werden zugegeben. Das Gemisch wird 6 Stunden bei einer Aussentemperatur von 150° erhitzt, abgekühlt und filtriert. Der Niederschlag wird mit Wasser gewaschen und im Hochvakuum getrocknet. Man erhält reines 9-Chlor-5-imino-2-(2-tetrahydrofuryl)-5,6-dihydro[1,2,4]triazolo[1,5-c]chinazolin, Fp. 203-206°.

Das Ausgangsmaterial wird wie folgt hergestellt: Eine Mischung von 45 ml Phosphorylchlorid, 0,3 g Phosphorpentachlorid und 2,1 g des Produkts von Beispiel 26 wird unter Stickstoff bei Raumtemperatur gerührt. Nach fünf Minuten werden 1,2 ml Pyridin zugetropft. Der Ansatz wird während einer Stunde allmählich auf 110° erhitzt, bei dieser Temperatur 16 Stunden gerührt, filtriert und unter vermindertem Druck zur Trockne eingeengt. Der Rückstand wird in Essigester aufgenommen, mit 2N Salzsäure gewaschen, über Natriumsulfat getrocknet und zur Trockne eingeengt. Umkristallisieren aus Essigester ergibt die Dichlor-Verbindung, die bei 150-152° schmilzt.

Beispiel 42: Ein Gemisch von 300 ml bei 0° mit Ammoniakgas gesättigter konzentrierter wässriger Ammoniumhydroxidlösung und 5,7 g 9-Chlor-2-(2-furyl)-5-methylthio[1,2,4]triazolo[1,5-c]chinazolin wird in einem Autoklaven aus rostfreiem Stahl bei einer Aussentemperatur von 150° 18 Stunden erhitzt. In dieser Zeit steigt der Druck auf 1,7 MPa. Nach Abkühlen wird das feste Material isoliert, mit Wasser gewaschen und an der Luft getrocknet. Das so erhaltene 9-Chlor-2-(2-furyl)-5-imino-5,6-dihydro[1,2,4]triazolo[1,5-c]-chinazolin, Fp. 269-271°, wird in das Methansulfonatsalz übergeführt, indem es in 150 ml 2-Methoxyethanol gelöst und mit 0,66 ml Methansulfonsäure und 25 ml trockenem Ether versetzt wird. Man erhält so das reine Salz, das identisch mit dem des Beispieles 32 ist.

Das Ausgangsmaterial wird wie folgt hergestellt: Eine Suspension von 40,6 g 2-Amino-5-chlorbenzonitril in 400 ml Wasser wird unter Stickstoff und unter kräftigem Rühren tropfenweise mit 20,3 ml Thiophosgen versetzt. Nach dreistündigem Rühren wird das Isothiocyanat isoliert, mit Wasser gewaschen, dann zweimal mit je 100 ml Cyclohexan, und im Vakuum getrocknet. 18,5 g des Isothiocyanats und 28,7 g 2-Furoylhydrazin in Dioxan werden unter Stickstoff eine Stunde unter Rückfluss erhitzt. Nach dem Abkühlen werden 22,2 ml Triethylamin und 0,1 ml Methansulfonsäure zugesetzt. Nach zwei weiteren Stunden unter Rückfluss wird der Ansatz unter vermindertem Druck zur Trockne eingeengt, in 300 ml Isopropanol suspendiert und eine Stunde kräftig gerührt. Der Feststoff wird isoliert und an der Luft getrocknet. Das so erhaltene 9-Chlor-2-(2-furyl)-5-thiono-5,6-dihydro[1,2,4]triazolo[1,5-c]chinazolin wird direkt in die nächste Stufe eingesetzt:

Ein Gemisch von 17,7 g 9-Chlor-2-(2-furyl)-5-thiono-5,6-dihydro-[1,2,4]triazolo[1,5-c]chinazolin, 200 ml Methanol und 31,4 g Natriummethanolat wird fünf Minuten bei Raumtemperatur unter Stickstoff gerührt und dann tropfenweise mit 3,7 ml Methyliodid versetzt. 200 ml Methanol werden zugegeben, der Ansatz wird unter Stickstoff zwei Stunden bei einer Badtemperatur von 80° gerührt, abgekühlt, und das 9-Chlor-2-(2-furyl)-5-methylthio[1,2,4]triazolo-[1,5-c]chinazolin wird isoliert, mit Wasser gewaschen und an der Luft getrocknet, Fp. 202-207°.

Beispiel 43: Analog zum Beispiel 42 wird 2-[2-(N-Methylpiperidyl)]-5-methylthio[1,2,4]triazolo[1,5-c]chinazolin in 5-Imino-2-[2-(N-methylpiperidyl)]-5,6-dihydro[1,2,4]triazolo[1,5-c]chinazolin, Fp. 193-194°, übergeführt. Die Methylthio-Verbindung, Fp. 142°, wird aus der Mercapto-Verbindung hergestellt, die sich wiederum aus o-Cyanphenylisothiocyanat und N-Methylpipecolinsäurehydrazid, wie in Beispiel 42 beschrieben, gewinnen lässt.

Beispiel 44: Analog zu Beispiel 42 wird 2-[2-(N-Methylpyrrolyl)]-5-methylthio[1,2,4]triazolo[1,5-c]chinazolin in 5-Imino-2-[2-(N-methylpyrrolyl)]-5,6-dihydro[1,2,4]triazolo[1,5-c]chinazolin, Fp. 251-254°, übergeführt. Das Ausgangsmaterial wird, wie in Beispiel 42 beschrieben, hergestellt, indem man von o-Cyanphenylisothiocyanat und N-Methylpyrrol-2-carbonsäurehydrazid ausgeht.

Beispiel 45: 4,28 g 2-(2-Pyrrolyl)-5-thiono-5,6-dihydro[1,2,4]-triazolo[1,5-c]chinazolin, analog zu Beispiel 39 hergestellt aus 5-Chlor-2-isothiocyanatobenzonitril und 2-Pyrrolcarbohydrazid, werden mit 225 ml einer 40 % wässrigen Lösung von Methylamin, die bei 0° mit Methylamingas gesättigt wurde, in einen Autoklaven aus rostfreiem Stahl gegeben und 6 Stunden bei einer Aussentemperatur von 150° erhitzt. Der Ansatz wird auf Raumtemperatur abgekühlt, der Feststoff wird isoliert, mit Wasser gewaschen und an der Luft getrocknet. Umkristallisieren aus wässrigem Methanol, dann aus Wasser ergibt reines 5-Methylimino-5,6-dihydro-2-(2-pyrrolyl)-[1,2,4]triazolo[1,5-c]chinazolin, Fp. 220-221°.

Beispiel 46: Analog zu Beispiel 39 wird 3-Furancarbonsäurehydrazid mit 5-Chlor-2-cyanphenylisothiocyanat umgesetzt. Man erhält 2-(3-Furyl)-5-mercapto[1,2,4]triazolo[1,5-c]chinazolin, Fp. >300°, das in die 5-Methylthio-Verbindung übergeführt wird und darauf in das 9-Chlor-2-(3-furyl)-5-imino-5,6-dihydro[1,2,4]triazolo-[1,5-c]chinazolin, das als Methansulfonatsalz gereinigt wird, Fp. 290-292°.

Beispiel 47: Analog zu Beispiel 13 wird 3-(Amino-5-chlorphenyl)-5-(3-furyl)-1H-1,2,4-triazol, das durch Eindampfen der Mutterlauge aus der Herstellung der Imino-Verbindung des Beispiels 46 erhalten wird, in das 9-Chlor-2-(3-furyl)[1,2,4]triazolo[1,5-c]chinazolin-5(6H)on, übergeführt, das nach Umkristallisieren aus Dimethylacetamid-Wasser einen Fp. >350° aufweist.

Beispiel 48: Ein Gemisch von 5,7 g 9-Chlor-2-(2-furyl)-5-methylthio[1,2,4]triazolo[1,5-c]chinazolin und 40 ml Cyclohexylamin wird 6 Stunden unter Stickstoff unter Rückfluss erhitzt. Nach Abkühlen wird der Feststoff isoliert und aus Ethanol umkristallisiert. Man erhält reines 9-Chlor-5-cyclohexylamino-2-(2-furyl)[1,2,4]triazolo[1,5-c]chinazolin, Fp. 158-160°.

Beispiel 49: Ein Gemisch von 5,6 g 9-Chlor-2-(2-furyl)-5-mercapto-[1,2,4]triazolo[1,5-c]chinazolin und 40 ml Anilin wird unter Stickstoff 66 Stunden bei 150° gerührt. Das Gemisch wird am Rotationsverdampfer unter Wasserstrahlvakuum erhitzt, um restliches Anilin zu entfernen und aus wässrigem Ethanol umkristallisiert. Man erhält reines 9-Chlor-2-(2-furyl)-5-phenylimino-5,6-dihydro[1,2,4]-triazolo[1,5-c]chinazolin, Fp. 231-233°.

Beispiel 50: Analog zu Beispiel 37 werden 1,06 g 9-Chlor-2-(2-furyl)-5-methylthio[1,2,4]triazolo[1,5-c]chinazolin in einem Autoklaven aus rostfreiem Stahl mit 50 ml Isopropylamin bei 150° (Aussentemperatur) während 6 Stunden zur Reaktion gebracht. Man

erhält 9-Chlor-2-(2-furyl)-5-isopropylimino-5,6-dihydro[1,2,4]-triazolo[1,5-c]chinazolin, das nach Umkristallisieren aus Ethanol bei 140-141° schmilzt.

Beispiel 51: Ein Gemisch von 1,9 g N-Carbethoxyanthranilonitril, hergestellt nach Breukink und Verkade, Rec. Trav. Chim. 79, 443 (1960), sowie 1,26 g 2-Furancarbonsäurehydrazid, 50 ml 2-Methoxyethanol und 1 ml Tri-n-propylamin wird unter Stickstoff 20 Stunden unter Rückfluss erhitzt. Ein Teil des Lösungsmittels, 30 ml, wird abdestilliert, man lässt die Mischung abkühlen. Die weissen Kristalle werden isoliert, mit Methanol gewaschen und im Vakuum getrocknet. Man erhält reines 2-(2-Furyl)[1,2,4]triazolo-[1,5-c]chinazolin-5(6H)on, Fp. 338-343°.

Beispiel 52: Analog zu Beispiel 51 wird 9-Chlor-2-(3-chlorphenyl)-[1,2,4]triazolo[1,5-c]chinazolin-5(6H)on, Fp. >350°, aus 4,2 g N-Carbomethoxy-5-chlor-2-aminobenzonitril und 3,4 g 3-Chlorbenzhydrazid hergestellt. Das verwendete Urethan-Ausgangsmaterial wird hergestellt, indem man 15,6 g 2-Amino-5-chlorbenzonitril, 200 ml Ethylmethylketon und 10 g Natriumbicarbonat mit 8,4 ml Chlorkohlensäuremethylester 42 Stunden unter Stickstoff bei 80° reagieren lässt. Der Ansatz wird abgekühlt, durch Filtrieren von anorganischem Material befreit, unter vermindertem Druck zur Trockne eingeengt und aus Chlorbenzol-Cyclohexan umkristallisiert; das so erhaltene Produkt weist einen Fp. von 126-132° auf.

Beispiel 53: Auf zu Beispiel 52 analoge Weise werden 4,2 g N-Carbo-methoxy-5-chlor-2-aminobenzonitril mit 3,1 g 2-Fluorbenzhydrazid in 100 ml 2-Methoxyethanol mit 0,3 ml Tri-n-propylamin unter Stickstoff 24 Stunden unter Rückfluss erhitzt. Man erhält reines 9-Chlor-2-(2-fluorphenyl)[1,2,4]triazolo[1,5-c]chinazolin-5(6H)on, das mit dem in Beispiel 22 beschriebenen Material identisch ist.

Beispiel 54: Ein Gemisch von 3,0 g N-(2-Cyanphenyl)-O-ethylthio-urethan, 1,83 g 2-Furancarbonsäurehydrazid, 2 ml Tri-n-propylamin und 50 ml 2-Methoxyethanol wird 18 Stunden unter Stickstoff und

Rückfluss erhitzt. Der Ansatz wird unter vermindertem Druck zu einem zähen Oel eingeengt, das bei Behandeln mit Methanol kristallisiert. Der Feststoff ist identisch mit dem 2-(2-Furyl)-5-mercapto[1,2,4]-triazolo[1,5-c]chinazolin, dessen Herstellung in Beispiel 37 beschrieben ist.

Das Thiourethan wird hergestellt, indem o-Cyanphenylisothiocyanat in überschüssigem absolutem Ethanol über Nacht unter Stickstoff unter Rückfluss erhitzt wird. Nach Filtrieren bei Raumtemperatur wird das Filtrat unter vermindertem Druck zur Trockne eingeengt. Der verbleibende Feststoff wird aus Cyclohexan umkristallisiert, man erhält so das Thiourethan, Fp. 87-89°.

Beispiel 55: Unter Kühlen in einem Eisbad wird während 90 Minuten Ammoniakgas durch eine Lösung von 15 g 9-Chlor-2-(2-furyl)-5-thiocyanato[1,2,4]triazolo[1,5-c]chinazolin in 200 ml 1,3-Dimethylimidazolidon geleitet. Nach einer weiteren Stunde bei Umgebungstemperatur wird der Ansatz mit 500 ml Wasser verdünnt, der Feststoff isoliert und aus 2-Methoxyethanol unter Zugabe einer kleinen Menge Wasser umkristallisiert. Nach 18stündigem Trocknen bei 100°/26 Pa wird reines 9-Chlor-2-(2-furyl)-5-imino-5,6-dihydro[1,2,4]triazolo-[1,5-c]chinazolin, Fp. 279-280°, erhalten.

Das Ausgangsmaterial wird, prinzipiell wie von Vlattas et al., J. Heterocyclic Chem. 20, 1287 (1983) beschrieben, wie folgt hergestellt: In einer Stickstoff-Atmosphäre werden zu einer Suspension von 790 mg Natriumhydrid (50%ig in Oel) in 40 ml trockenem Tetrahydrofuran 5 g 9-Chlor-2-(2-furyl)-5-thiono-5,6-dihydro-[1,2,4]triazolo[1,5-c]chinazolin portionsweise gegeben. Der Ansatz wird zwei Stunden unter Stickstoff gerührt, äusserlich auf 0° abgekühlt und unter Stickstoff tropfenweise mit einer Lösung von 1,75 g Bromcyan in 15 ml Tetrahydrofuran versetzt. Er wird im Eisbad 90 Minuten gerührt, mit 100 ml Wasser verdünnt und dreimal mit je 100 ml Essigester extrahiert. Die vereinigten organischen Extrakte werden filtriert, über Magnesiumsulfat getrocknet und unter vermindertem Druck zur Trockne eingeengt. Man erhält so die gewünschte Thiocyanat-Verbindung in genügender Reinheit. Umkristallisieren aus 2-Methoxyethanol führt zum reinen Stoff, Fp. 218-220°.

Beispiel 56: Ein Gemisch von 1,79 g N,N-Dimethyl-N'-(4-chlor-2-cyanphenyl)harnstoff, 1,05 g 2-Furancarbonsäurehydrazid und 25 ml 2-Methoxyethanol wird 20 Stunden unter Stickstoff unter Rückfluss erhitzt. Das Gemisch wird abgekühlt und das kristalline 9-Chlor-2-(2-furyl)[1,2,4]triazolo[1,5-c]chinazolin-5(6)on isoliert, mit Wasser, danach mit Methanol gewaschen und im Vakuum getrocknet. Das Material ist identisch mit der nach Beispiel 18 erhaltenen Verbindung.

Der als Ausgangsstoff verwendete Harnstoff wird hergestellt, indem 2,7 g 4-Chlor-2-cyanphenylisocyanat in 100 ml warmem Toluol gelöst und mit 20 ml einer 17,6%igen Lösung von Dimethylamin in Toluol 20 Stunden bei Raumtemperatur unter Stickstoff behandelt werden. Der Ansatz wird unter vermindertem Druck zur Trockne eingeengt, wobei man die Temperatur auf nicht mehr als 50° steigen lässt. Man erhält einen Feststoff, der durch Umkristallisieren aus Cyclohexan in den reinen Harnstoff, Fp. 95-97°, übergeführt wird.

Beispiel 57: Ein Gemisch von 2,15 g N-Carbethoxy-5-nitroanthranilonitril, 1,56 g 2-Fluorbenzhydrazid, 1 ml Tri-n-propylamin und 50 ml (RS)-1-Methoxy-2-propanol wird 20 Stunden unter Stickstoff unter Rückfluss erhitzt. Der Ansatz wird abgekühlt, mit 50 ml Methanol behandelt und filtriert. Der weisse Niederschlag wird mit Methanol gewaschen und aus Dimethylacetamid/Ethanol umkristallisiert. Man erhält so reines 2-(2-Fluorphenyl)-9-nitro[1,2,4]triazolo[1,5-c]-chinazolin-5(6H)-on, das oberhalb von 350° schmilzt.

Beispiel 58: Eine Lösung von 2 g 9-Chlor-2-(2-furyl)-5-thiocyanato-[1,2,4]triazolo[1,5-c]chinazolin in 28 ml 1,3-Dimethylimidazolidon wird zu 2 ml tert-Butylamin gegeben. Das Gemisch wird 3 Stunden unter Stickstoff gerührt, mit 20 ml Ethanol verdünnt und mit 20 ml Wasser versetzt. Der Niederschlag wird isoliert, aus 2-Methoxyethanol umkristallisiert und im Hochvakuum getrocknet. Man erhält so reines 5-tert-Butylamino-9-chlor-2-(2-furyl)[1,2,4]triazolo[1,5-c]-chinazolin, Fp. >330°.

Beispiel 59: Wenn statt tert-Butylamin, wie in Beispiel 58, Ethanolamin verwendet wird, wird auf analoge Weise 9-Chlor-2-(2-furyl)-5-hydroxyethylamino[1,2,4]triazolo[1,5-c]chinazolin, Fp. 214-217°, erhalten.

Beispiel 60: Zu einer Lösung von 1,45 g 5,9-Dichlor-2-(2-furyl)-[1,2,4]triazolo[1,5-c]chinazolin in 150 ml trockenem Tetrahydrofuran wird bei 50° während 40 Minuten gasförmiges Methylamin gegeben. Das Gemisch wird zur Trockne eingeengt, mit Wasser behandelt und filtriert. Man erhält so einen weissen Feststoff, der zum Umkristallisieren in heissem Toluol aufgenommen wird. Nachdem man einige Minuten erhitzt hat, um Wasser durch azeotropes Abdestillieren zu entfernen, wird beim Abkühlen Cyclohexan zugesetzt. Auf diese Weise wird reines 9-Chlor-2-(2-furyl)-5-methylamino[1,2,4]triazolo[1,5-c]-chinazolin, Fp. 166-168°, erhalten.

Das Ausgangsmaterial wird wie folgt erhalten: Ein Gemisch von 15 g 9-Chlor-2-(2-furyl)[1,2,4]triazolo[1,5-c]chinazolin-5(6H)on (Beispiel 18) und 275 ml P,P-Dichlorphenylphosphinoxid wird 24 Stunden auf 182 bis 187° erhitzt. Ueberschüssiges Lösungsmittel wird im Vakuum abgezogen, der Rückstand wird abgekühlt und mit 750 ml Dichlormethan behandelt. Es wird unter Rückfluss erhitzt, restliches Ausgangsmaterial wird abfiltriert. Das Filtrat wird mit Aktivkohle behandelt, über Diatomeenerde filtriert und unter vermindertem Druck auf ein kleines Volumen eingeengt. Die Dichlor-Verbindung kristallisiert bei 0°, Fp. 242-242,5° (Z).

Beispiel 61: Wenn die vorstehend beschriebene 5,9-Dichlor-Verbindung unter Eiskühlung mit überschüssiger konzentrierter Ammoniumhydroxidlösung umgesetzt wird und man den Ansatz mit Wasser verdünnt, wird 9-Chlor-2-(2-furyl)-5-imino-5,6-dihydro[1,2,4]triazolo[1,5-c]-chinazolin erhalten, Fp. 276-278°.

Beispiel 62: Zu einer Lösung von 4,9 g 2-(5-Brom-2-furyl)-9-chlor-5-thiocyanato[1,2,4]triazolo[1,5-c]chinazolin in 80 ml Tetrahydrofuran wird während 1,3 Stunden unter Eiskühlung gasförmiges Ammoniak gegeben. Der sich bildende Niederschlag wird isoliert, mit Tetrahydrofuran gewaschen, aus N,N-Dimethylacetamid umkristallisiert und im Hochvakuum bei 100° getrocknet. Man erhält so reines 2-(5-Brom-2-furyl)-9-chlor-5-imino-5,6-dihydro[1,2,4]triazolo[1,5-c]-chinazolin, Fp. 282-283°.

Das Ausgangsmaterial wird wie folgt hergestellt: Ein Gemisch von 6 g 5-Brom-2-furancarbonsäurehydrazid (hergestellt nach Blomquist und Stevenson, J. Am. Chem. Soc. 56, 148 (1934)), 5,8 g 5-Chlor-2-cyanphenylisothiocyanat und 70 ml N-Methylpyrrolidon wird bei 150° 4 Stunden unter Stickstoff gerührt und in eine Mischung aus 100 ml Eiswasser und 100 ml Isopropanol gegeben. Der Niederschlag wird isoliert und auf dem Filter so trocken gepresst wie möglich. Er wird in 150 ml heissem Dioxan gelöst, filtriert, auf 20 ml eingeengt, abgekühlt und mit 100 ml Methanol behandelt. Man erhält so reines 2-(5-Brom-2-furyl)-9-chlor-5-mercapto[1,2,4]triazolo[1,5-c]-chinazolin, Fp. 273-276°. Die 5-Mercapto-Verbindung wird dann in die 5-Thiocyanato-Verbindung übergeführt, wie es in Beispiel 55 beschrieben ist.

Beispiel 63: Zu einer Mischung von 15,1 g 3-(2-Amino-5-chlorphenyl)-5-(2-furyl)-1,2,4-triazol (Zwischenstufe aus Beispiel 18) und 300 ml Isopropanol werden unter Rühren 7,4 ml einer 50 %-igen wässrigen Cyanamidlösung gegeben, danach ein Gemisch von 3,7 g konzentrierter Schwefelsäure und 4 ml Wasser. Die Mischung wird 6 Stunden unter Rückfluss erhitzt, auf Raumtemperatur abgekühlt, und der pH wird durch Zugabe von 10 %-iger Natronlauge auf 7 eingestellt. Die Mischung wird in einem Eisbad gekühlt und eine halbe Stunde bei 5° gerührt. Der Niederschlag wird isoliert, mit kaltem wasserfreien Ethanol gewaschen und 18 Stunden im Vakuum bei 80° getrocknet. Das Rohprodukt wird bei 110-115° in 240 ml Eisessig gelöst, mit Aktivkohle behandelt und heiss durch Diatomeenerde

filtriert. Das Filtrat wird auf Raumtemperatur abgekühlt, eine Stunde gerührt, und der Feststoff isoliert, mit drei 80 ml Portionen Wasser, danach mit 40 ml wasserfreiem Ethanol gewaschen und im Vakuum bei 80° 18 Stunden getrocknet. Man erhält so reines 9-Chlor-2-(2-furyl)-5-imino-5,6-dihydro[1,2,4]triazolo[1,5-c]chinazolin, identisch mit dem Produkt von Beispiel 61.

Das Ausgangsmaterial lässt sich abweichend von der in Beispiel 13 beschriebenen Weise auch wie folgt herstellen: Unter heftigem Rühren wird durch eine Lösung von 47,55 g 2-(Ethoxycarbonylamino)benzonitril (siehe Beispiel 51) in 100 ml N,N-Dimethylformamid soviel Chlorgas geleitet, dass die Innentemperatur bei 30° ± 2° bleibt. Wenn gemäss HPLC-Analyse kein Ausgangsmaterial mehr vorhanden ist, wird unter weiterem Rühren während einer Stunde Stickstoff durch den Ansatz geleitet, dann werden 200 ml Wasser tropfenweise unter Kühlen zugegeben. Das Produkt wird isoliert, mit Wasser gewaschen und bei 50-60° 16 Stunden im Vakuum getrocknet. Das erhaltene 5-Chlor-2--(ethoxycarbonylamino)benzonitril, Fp. 126-129° wird aus wässrigem Ethanol umkristallisiert, man erhält so reines Material vom Fp. 131-133°. 15 g dieses Urethans werden zusammen mit 8,42 g 2-Furancarbonsäurehydrazid in 75 ml N-Methylpyrrolidon während 5 Stunden in einer Apparatur mit Wasserabscheider bei einer Badtemperatur von 160° gerührt. Ein Teil des Lösungsmittels destilliert über, die Lösung wird abgekühlt, und 150 ml Wasser werden allmählich zugegeben, wenn die Temperatur sinkt. Das Produkt wird bei Raumtemperatur isoliert, mit 100 ml Wasser, danach mit 50 ml Isopropanol gewaschen und 16 Stunden im Vakuum bei 70-75° getrocknet. Das Produkt, 9-Chlor-2-(2-furyl)[1,2,4]triazolo[1,5-c]chinazolin-5-(6H)on, ist identisch mit dem von Beispiel 18. 60 g dieses Produktes werden in 350 ml Ethylenglykol gerührt und mit einer Lösung von 17,1 g Natriumhydroxid in 55 ml Wasser versetzt. Der Ansatz wird 19 Stunden unter Rückfluss gerührt, auf Raumtemperatur abgekühlt und mit 550 ml Wasser behandelt. Der pH-Wert wird danach durch Zugabe von Eisessig auf 6,5 bis 7,0 eingestellt, es wird weitere 15 Minuten gerührt, der Feststoff wird isoliert, mit drei 50 ml Portionen Wasser gewaschen und 16 Stunden im Vakuum bei 80° getrocknet. Man

erhält so reines 3-(2-Amino-5-chlorphenyl)-5-(2-furyl)-1,2,4-triazol, das mit der in Beispiel 18 verwendeten Zwischenstufe identisch ist.

Beispiel 64: Herstellung von 10 000 Tabletten, die jeweils 50 mg Wirkstoff enthalten, mit folgender Zusammensetzung:

| | |
|---|---|
| 2-(2-Fluorphenyl)-9-chlor[1,2,4]-triazolo[1,5-c]chinazolin-5(6H)on | 500 g |
| Milchzucker | 707 g |
| Maisstärke | 75 g |
| Polyethylenglykol 6000 | 75 g |
| Talkum | 75 g |
| Magnesiumstearat | 18 g |
| gereinigtes Wasser | q.s. |

Verfahren: Sämtliche pulvrigen Bestandteile werden mit einem Sieb von 0,6 mm Maschenweite gesiebt. Dann wird der Wirkstoff mit Milchzucker, Talkum, Magnesiumstearat und mit der Hälfte der Stärke in einem geeigneten Mischer vermischt. Die andere Hälfte der Stärke wird in 40 ml Wasser suspendiert, und die Suspension wird zur siedenden Lösung von Polyethylenglykol in 150 ml Wasser gegeben. Die erhaltene Paste wird zu den Pulvern gegeben und, gegebenenfalls unter Zugabe einer weiteren Wassermenge, granuliert. Das Granulat wird über Nacht bei 35° getrocknet, durch ein Sieb von 1,2 mm Maschenweise getrieben und zu Tabletten gepresst, die eine Bruchrille aufweisen.

Beispiel 65: Herstellung von 10'000 Kapseln mit einem Gehalt von jeweils 20 mg der aktiven Substanz mit folgender Zusammensetzung:

| | |
|---|---|
| 2-(3-Fluorphenyl)-9-chlor[1,2,4]-triazolo[1,5-c]chinazolin-5(6H)on | 200 g |
| Milchzucker | 1700 g |
| Modifizierte Stärke | 100 g |

Verfahren: Sämtliche pulvrigen Bestandteile werden mit einem Sieb von 0,6 mm Maschenweite gesiebt. Dann wird der Wirkstoff in einen geeigneten Mischer gegeben und bis zur Homogenität zuerst mit der Stärke, dann mit Milchzucker vermischt. No. 3 Gelatinekapseln werden mit je 200 mg dieser Mischung gefüllt, wobei man eine Kapselabfüllmaschine verwendet.

Auf analoge Weise werden Kapseln oder Tabletten hergestellt, die 10-200 mg der anderen offenbarten und in den Beispielen erwähnten Verbindungen enthalten.

Patentansprüche

1. Verbindungen der Formel I,

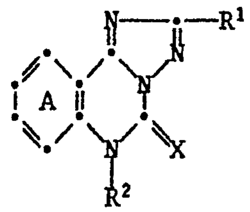

(I)

worin $R^1$ Phenyl oder durch Niederalkyl, Niederalkoxy, Hydroxy, Halogen oder Trifluormethyl substituiertes Phenyl bedeutet, oder einen 5-gliedrigen aromatischen oder teilweise oder vollständig gesättigten heterocyclischen Ring, der 1 bis 3 Heteroatome aus der Gruppe N, O und S enthält oder einen 6-gliedrigen aromatischen oder teilweise oder vollständig gesättigten heterocyclischen Ring, der 1 bis 4 Heteroatome aus der Gruppe N, O und S enthält, wobei dieser heterocyclische Ring an den Triazolochinazolinkern über ein Kohlenstoffatom gebunden ist, und wobei dieser heterocyclische Ring unsubstituiert oder durch Niederalkyl, Hydroxy, Amino, Halogen oder Hydroxyniederalkyl substituiert sein kann, $R^2$ Wasserstoff, Niederalkyl, Hydroxyniederalkyl, Niederalkenyl, Arylniederalkyl, Arylniederalkenyl oder Aryl bedeutet, Ring A unsubstituiert oder durch Niederalkyl, Niederalkoxy, Hydroxy, Halogen, Trifluormethyl, Nitro, Amino, Niederalkylthio, Niederalkylsulfinyl, Niederalkylsulfonyl oder Arylniederalkoxy substituiert ist, und worin X Sauerstoff, Schwefel oder N-$R^3$ bedeutet, worin $R^3$ für Wasserstoff, Niederalkyl, Arylniederalkyl, Cycloalkyl, Niederalkenyl, worin die Doppelbindung vom Stickstoffatom durch mindestens ein gesättigtes Kohlenstoffatom getrennt ist, Niederalkinyl, worin die Dreifachbindung vom Stickstoffatom durch mindestens ein gesättigtes Kohlenstoffatom getrennt ist, Aryl, Aminoniederalkyl, Niederalkylaminoniederalkyl, Diniederalkylaminoniederalkyl oder Hydroxyniederalkyl steht, sowie Salze und Tautomere dieser Verbindungen.

2. Verbindungen der Formel I nach Anspruch 1, worin $R^1$ für Phenyl steht oder für Phenyl, das durch 1 bis 3 Reste der Art Niederalkyl, Niederalkoxy, Hydroxy, Halogen oder Trifluormethyl substituiert ist; oder $R^1$ bedeutet einen 5-gliedrigen aromatischen oder teilweise oder vollständig gesättigten heterocyclischen Ring, der 1 bis 3 Heteroatome aus der Gruppe N, O und S enthält oder einen 6-gliedrigen aromatischen oder teilweise oder vollständig gesättigten heterocyclischen Ring, der 1 bis 4 Heteroatome aus der Gruppe N, O und S enthält, wobei dieser heterocyclische Ring an den Triazolochinazolinkern über ein Kohlenstoffatom gebunden ist, und wobei dieser heterocyclische Ring unsubstituiert oder durch Niederalkyl, Hydroxy oder Halogen substituiert sein kann; worin $R^2$ für Wasserstoff, Niederalkyl, Arylniederalkyl, Niederalkenyl, Arylniederalkenyl oder Aryl steht, worin X O, S oder N-$R^3$ bedeutet, wobei $R^3$ für Wasserstoff, Niederalkyl, Arylniederalkyl, Aminoniederalkyl oder Niederalkylaminoniederalkyl steht; und worin Ring A unsubstituiert ist oder durch ein bis drei Gruppen der Art Niederalkyl, Niederalkoxy, Hydroxy, Halogen oder Trifluormethyl substituiert ist; und pharmazeutisch annehmbare Salze davon.

3. Verbindungen der Formel I nach Anspruch 1, worin $R^1$ für Phenyl steht oder für Phenyl, das durch ein bis drei Reste der Art Niederalkyl, Niederalkoxy, Hydroxy, Halogen und Trifluormethyl substituiert ist; oder für einen 5-gliedrigen aromatischen heterocyclischen Ring, der 1 bis 3 Heteroatome aus der Gruppe N, O und S enthält oder für einen 6-gliedrigen aromatischen heterocyclischen Ring, der 1 bis 4 Heteroatome aus der Gruppe N, O und S enthält, wobei ein solcher heterocyclischer Ring über ein Kohlenstoffatom gebunden ist; wobei dieser aromatische heterocyclische Ring unsubstituiert oder durch Hydroxy, Niederalkyl oder Halogen substituiert sein kann; worin $R^2$ für Wasserstoff oder Niederalkyl steht; worin X Sauerstoff oder N-$R^3$ bedeutet, worin $R^3$ für Wasserstoff oder Niederalkyl steht, und worin Ring A unsubstituiert ist oder substituiert durch 1 bis 3 Gruppen der Art Halogen oder Niederalkyl, sowie pharmazeutisch annehmbare Salze davon.

4. Verbindungen der Formel I nach Anspruch 1, worin $R^1$ für Phenyl steht oder für Phenyl, das durch ein bis drei Reste der Art Niederalkyl, Niederalkoxy, Hydroxy, Halogen und Trifluormethyl substituiert ist, worin $R^2$ für Wasserstoff oder Niederalkyl steht, X Sauerstoff bedeutet, und worin Ring A unsubstituiert ist oder substituiert durch 1 bis 3 Gruppen der Art Halogen oder Niederalkyl, sowie pharmazeutisch annehmbare Salze davon.

5. Verbindungen der Formel I nach Anspruch 1, worin $R^1$ für einen 5-gliedrigen aromatischen heterocyclischen Ring, der 1 bis 3 Heteroatome aus der Gruppe N, O und S enthält oder für einen 6-gliedrigen aromatischen heterocyclischen Ring, der 1 bis 4 Heteroatome aus der Gruppe N, O und S enthält, steht, wobei ein solcher heterocyclischer Ring über ein Kohlenstoffatom gebunden ist; wobei dieser aromatische heterocyclische Ring unsubstituiert oder durch Hydroxy, Niederalkyl, oder Halogen substituiert sein kann; worin $R^2$ für Wasserstoff oder Niederalkyl steht; X $N-R^3$ bedeutet, worin $R^3$ für Wasserstoff oder Niederalkyl steht, und worin Ring A unsubstituiert ist oder substituiert durch 1 bis 3 Gruppen der Art Halogen oder Niederalkyl, sowie pharmazeutisch annehmbare Salze davon.

6. Verbindungen der Formel I nach Anspruch 1, worin $R^1$ für Phenyl oder für durch Halogen substituiertes Phenyl steht, oder für Furyl; worin $R^2$ Wasserstoff bedeutet; worin X für Sauerstoff oder für NH steht, und worin Ring A durch Halogen in der 8- oder 9-Stellung substituiert ist, und pharmazeutisch annehmbare Salze davon.

7. Verbindungen der Formel I nach Anspruch 1, worin $R^1$ für durch Halogen substituiertes Phenyl steht, $R^2$ Wasserstoff bedeutet, X für Sauerstoff steht und Ring A in der 8- oder 9-Stellung durch Halogen substituiert ist, und pharmazeutisch annehmbare Salze davon.

8. Verbindungen der Formel I nach Anspruch 1, worin $R^1$ für Furyl steht, $R^2$ Wasserstoff bedeutet, X für NH steht und Ring A in der 8- oder 9-Stellung durch Halogen substituiert ist, und pharmazeutisch annehmbare Salze davon.

9. 9-Chlor-2-(2-fluorphenyl)[1,2,4]triazolo[1,5-c]chinazolin-5(6H)on und seine pharmazeutisch annehmbaren Salze.

10. 9-Chlor-2-(3-fluorphenyl)[1,2,4]triazolo[1,5-c]chinazolin-5(6H)on und seine pharmazeutisch annehmbaren Salze.

11. 9-Chlor-2-(2-furyl)-5-imino-5,6-dihydro[1,2,4]triazolo[1,5-c]-chinazolin und seine pharmazeutisch annehmbaren Salze.

12. Pharmazeutische Präparate enthaltend Verbindungen der Ansprüche 1 bis 11 zusammen mit einem pharmazeutisch geeigneten Trägermaterial.

13. Die in den Ansprüchen 1 bis 11 genannten Verbindungen zur Anwendung in einem Verfahren zur therapeutischen Behandlung des menschlichen oder tierischen Körpers.

14. Die in den Ansprüchen 1 bis 11 genannten Verbindungen als Anxiomodulatoren.

15. Die in den Ansprüchen 1 bis 11 genannten Verbindungen als Benzodiazepinantagonisten.

16. Verwendung der in den Ansprüchen 1 bis 11 genannten Verbindungen zur Herstellung von pharmazeutischen Präparaten.

17. Verwendung einer Verbindung der Formel I nach Anspruch 1 zur Herstellung eines pharmazeutischen Präparats zur Anwendung als Anxiomodulator.

18. Verfahren zur Herstellung eines pharmazeutischen Präparates, gekennzeichnet durch die Verarbeitung eines erfindungsgemässen Wirkstoffs nach einem der Ansprüche 1 bis 11 mit einem pharmazeutischen Trägermaterial.

19. Verfahren zur Herstellung von den in Anspruch 1 genannten Verbindungen der Formel I, in der alle Symbole die in Anspruch 1 angegebenen Bedeutungen haben, gekennzeichnet

a) durch Behandeln einer Verbindung der Formel II,

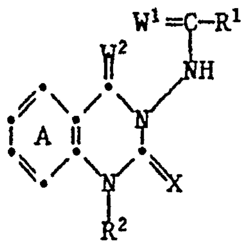

(II)

worin $R^1$, $R^2$, X und Ring A wie vorstehend definiert sind, einer der beiden Reste $W^1$ und $W^2$ für NH und der andere der beiden Reste $W^1$ und $W^2$ für O oder NH steht, mit einer Base, oder

b) durch Behandeln einer Verbindung der Formel III,

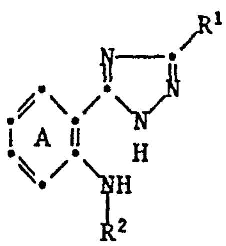

(III)

worin $R^1$, $R^2$ und Ring A wie vorstehend definiert sind, mit einem reaktiven Derivat der Kohlensäure, oder

c) durch Behandeln einer Verbindung der Formel IV,

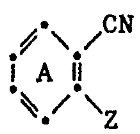

(IV)

worin Ring A wie vorstehend definiert ist und Z das Radikal eines Kohlensäurederivates, das über ein Stickstoffatom gebunden ist, bedeutet, mit einem Hydrazid der Formel V,

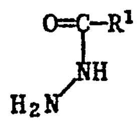

(V)

oder d), um eine Verbindung der Formel I, worin $R^2$ für Wasserstoff und X für Sauerstoff steht, zu erhalten, durch Behandeln einer Verbindung der Formel VI,

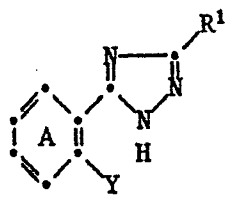

(VI)

worin Y für eine Gruppe steht, die sich durch ein Oxydationsmittel in die Gruppe -N=C=O überführen lässt, mit einem solchen Oxidationsmittel gefolgt von einem Ringschluss, oder

e), um eine Verbindung der Formel I zu erhalten, worin $R^2$ für Wasserstoff steht, durch Ueberführen der Gruppe L in einer Verbindung der Formel VII,

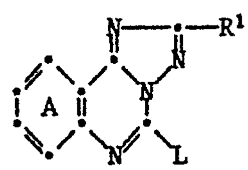

(VII)

worin L aus der Gruppe Halogen, Niederalkoxy, Arylniederalkoxy, Mercapto, Niederalkylthio und Isothiocyanato ausgewählt ist, in die Gruppe XH, oder,

f), um eine Verbindung der Formel I zu erhalten, worin $R^2$ für Wasserstoff steht, durch Behandeln einer Verbindung der Formel VIII

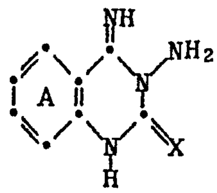

(VIII)

mit einem reaktiven Derivat einer Carbonsäure der Formel $R^1$-COOH, und wenn erwünscht, durch Ueberführung einer erhaltenen Verbindung in eine andere Verbindung der Erfindung und/oder durch Ueberführen eines erhaltenen Salzes in die freie Verbindung oder in ein anderes Salz und/oder durch Ueberführen einer erhaltenen freien Verbindung, die eine salzbildende Gruppe aufweist, in ein Salz.

20. Die nach dem Verfahren des Anspruchs 19 erhältlichen neuen Verbindungen.

FO 7.4/JL/cw*

Europäisches Patentamt

**0181282**

Nummer der Anmeldung

# EUROPÄISCHER RECHERCHENBERICHT

EP 85810438.3

| Kategorie | EINSCHLÄGIGE DOKUMENTE<br>Kennzeichnung des Dokuments mit Angabe, soweit erforderlich, der maßgeblichen Teile | Betrifft Anspruch | KLASSIFIKATION DER ANMELDUNG (Int. Cl 4) |
|---|---|---|---|
| A | EP - A1 - 0 080 176 (BASF AKTIEN-GESELLSCHAFT)<br>* Ansprüche 1,6,9,10; Seite 12 *<br>-- | 1-8,12, 13,16, 18-20 | C 07 D 487/04<br>A 61 K 31/505<br>A 61 K 31/41//<br>(C 07 D 487/04<br>C 07 D 249/00<br>C 07 D 239/00) |
| A | EP - A1 - 0 023 773 (E.R. SQIBB & SONS, INC.)<br>* Anspruch 1 *<br>---- | 19 | |
| | | | RECHERCHIERTE SACHGEBIETE (Int. Cl 4) |
| | | | C 07 D 487/00 |

Der vorliegende Recherchenbericht wurde für alle Patentansprüche erstellt.

| Recherchenort | Abschlußdatum der Recherche | Prüfer |
|---|---|---|
| WIEN | 06-12-1985 | PETROUSEK |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument